# EUROPEAN PATENT APPLICATION

(11) **EP 4 461 733 A1**
(43) Date of publication of application: **13.11.2024**
(21) Application number: 22918157.3
(22) Date of filing: 07.07.2022
(51) Int. Cl.: C07D 487/04, A61K 31/4985, A61P 35/00

(54) **1-AMINOBENZO[4,5]IMIDAZO[1,2-A]PYRAZINE-3-FORMAMIDE COMPOUND, AND PREPARATION THEREFOR AND USE THEREOF**

(30) Priority: 07.01.2022 CN 202210018141
(71) Applicant: Sun Yat-Sen University, Guangzhou, Guangdong 510275 (CN)
(72) Inventor: HU, Wenhao, Guangzhou, Guangdong 510006 (CN); DING, Wen, Guangzhou, Guangdong 510006 (CN); LIU, Shuhao, Guangzhou, Guangdong 510006 (CN); ZHANG, Zhijing, Guangzhou, Guangdong 510006 (CN); WU, Linna, Guangzhou, Guangdong 510006 (CN); HUANG, Weifeng, Guangzhou, Guangdong 510006 (CN); ZHANG, Xiaolei, Guangzhou, Guangdong 510006 (CN); SHI, Taoda, Guangzhou, Guangdong 510006 (CN); LEI, Jinping, Guangzhou, Guangdong 510006 (CN)
(74) Representative: Barker Brettell LLP
(86) International application number: PCT/CN2022/104398
(87) International publication number: WO 2023/130701

(57) **Abstract**

The present invention belongs to the technical field of pharmaceutical chemistry, and particularly relates to a 1-aminobenzo[4, 5]imidazo[1, 2-a]pyrazine-3-formamide compound, and the preparation therefor and use thereof. The structure of the compound of the present invention is as shown in formula (I). The compound has a novel structure and is an adenosine receptor inhibitor, has good inhibitory activity on adenosine A_{2A} receptors, has good subtype selectivity, and can be used as a targeted adenosine A_{2A} receptor inhibitor for immunotherapy. By means of inhibiting the activity of the adenosine A_{2A} receptor, the function of removing tumor cells by the immune system is released, so that the effect of treating tumors is achieved. Furthermore, the method for preparing the compound of the present invention is simple, uses cheap and easily available compounds as raw materials, has mild reaction conditions and high total yield and purity, can achieve dozens of grams of scale production, and supports the research of the clinical candidate adenosine A_{2A} receptor inhibitor in the later stage.

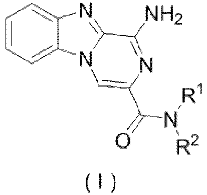

## Description

### Technical field

The present invention belongs to the technical field of pharmaceutical chemistry, and particularly relates to 1-aminobenzo [4, 5] imidazo [1, 2-a] pyrazine-3-formamide compound, and.preparation therefor and use therefor.

### Background of the invention

The adenosine receptor is a kind of G protein-coupled receptor, which contains seven transmembrane *α* helices, including four subtypes of A₁, A_{2A}, A_{2B} and A₃ receptors. There is a large amount of extracellular adenosine in the tumor microenvironment. High concentration of extracellular adenosine interacts with G protein-coupled adenosine receptors on the surface of the immune cell membrane. Adenosine transmits immunosuppressive signals into immune cells, thereby inhibits the proliferation and maturation of immune cells such as T cells, NK cells, macrophages, and dendritic DC cells. Adenosine also inhibits the production of immune cytokines (IL-2, IFN-γ), resulting in immune injury to the body, causing the immune escape of tumor cells. Among the four G protein-coupled adenosine receptors, adenosine A_{2A} is the most widely expressed subtype in T cells, NK cells and other immune cells, which produces immunosuppressive effects when combined with adenosine. Therefore, the development of adenosine receptor inhibitors is of great significance to improve the immune performance of the body.

At present, small molecule adenosine receptor inhibitors are being widely studied. They can improve the immune response of the body by blocking the transmission of immunosuppressive signals mediated by A_{2A} receptors. At present, many small molecule A_{2A} receptor inhibitors are in clinical research. These small molecule adenosine receptor inhibitors are often used in melanoma, lung squamous cell carcinoma, breast cancer, gastric cancer, ovarian cancer, prostate cancer and shows significant curative effect in these common tumor diseases.

However, the development of small molecule adenosine receptor inhibitors is still in infancy and requires more development. Therefore, there is an urgent need to develop more novel adenosine receptor inhibitors with anti-tumor activity.

### Summary of the invention

In order to overcome the shortcomings of the prior art, the primary purpose of the present invention is to provide a kind of 1-aminobenzo [4, 5] imidazo [1, 2-a] pyrazine-3-formamide compound.

The second purpose of the present invention is to provide a method for preparation of the above-mentioned 1-aminobenzo [4, 5] imidazo [1, 2-a] pyrazine-3-formamide compound.

The third purpose of the present invention is to provide the application of the above-mentioned 1-aminobenzo [4, 5] imidazo [1, 2-a] pyrazine-3-formamide compound. The compound has good inhibition activities on adenosine A_{2A} receptors and good subtype selectivity. They is expected to be used in tumor immunotherapy targeting adenosine A_{2A} receptor.

The said first purpose of the present invention is achieved by the following technical solution:
A kind of 1-aminobenzo [4, 5] imidazo [1, 2-a] pyrazine-3-formamide compound, the structure of the compound is shown in formula (I): 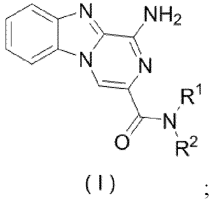
Wherein, R¹ is independently selected from hydrogen, C1-C4 alkyl, R² is independently selected from C1-C6 alkyl, C1-C4 alkanol group, C1-C4 haloalkyl, C1-C4 alkoxy, substituted phenyl, 2-pyridyl, 3-pyridyl, 4-pyridyl, 2-furyl, C4-C12 heterocycle, 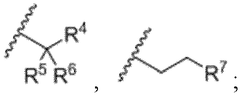
The said R⁴ is independently selected from substituted phenyl, 2-pyridyl, 3-pyridyl, 4-pyridyl, pyrimidinyl, pyrrolyl, imidazolyl, pyrazolyl, oxazolyl, pyrazinyl, pyridazinyl, 2-furyl, 2-substituted furyl, thienyl, 2-substituted thienyl, C6-C12 fused ring, C6-C12 N-hetero fused ring;
The said R⁵ is independently selected from hydrogen, deuterium, halogen, hydroxy, nitro, cyano, C1-C4 alkyl, C1-C4 haloalkyl, C1-C4 alkoxy, C3-C6 cycloalkyl;
The said R⁶ is independently selected from hydrogen, deuterium, halogen, hydroxy, nitro, cyano, C1-C4 alkyl, C1-C4 haloalkyl, C1-C4 alkoxy, C3-C6 cycloalkyl;
The said R⁷ is independently selected from substituted phenyl, 2-pyridyl, 3-pyridyl, 4-pyridyl, pyrimidinyl, pyrrolyl, imidazolyl, pyrazolyl, oxazolyl, pyrazine, pyridazinyl, 2-furyl, 2-substituted furyl, thienyl, 2-substituted thienyl, substituted indolyl, C6-C12 fused ring, C6-C12 N-hetero fused ring;
The said substituents in the 2-substituted furyl, substituted phenyl, and substituted indolyl are independently selected from C1-C4 alkyl, C1-C4 haloalkyl, C1-C4 alkoxy, C1-C4 alkanol group, cyano, halogen, hydroxyl and amino.

Preferably, the said R¹ is independently selected from hydrogen, methyl, ethyl, propyl, isopropyl, butyl; R² is independently selected from n-pentyl, isopentyl, n-butyl, isobutyl, 2-methoxyethyl, 2,2-difluoroethyl, 2-hydroxyethyl, 1-hydroxypropyl, 2-hydroxypropyl, 3-hydroxypropyl, 3-hydroxybutyl, 2-pyridyl, 3-pyridyl, 4-pyridyl, 2-furyl, 2-substituted furyl, thienyl, 2-substituted thienyl, substituted phenyl, 2-pyridyl, 3-pyridyl, 4-pyridyl, pyrimidinyl, pyrrolyl,, imidazolyl, pyrazolyl, oxazolyl, pyrazinyl, pyridazinyl, quinolyl, isoquinolyl, naphthyl, indolyl, substitute indolyl, 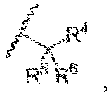 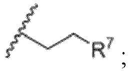
The said R⁴ is independently selected from substituted phenyl, 2-furyl, 2-substituted furyl, thienyl, 2-substituted thienyl, 2-pyridyl, 3-pyridyl, 4-pyridyl, pyrimidinyl, pyrrolyl, imidazolyl, pyrazolyl, oxazolyl, pyrazinyl, pyridazinyl, quinolyl, isoquinolyl, naphthyl, indolyl, substituted indolyl;
R⁵ is independently selected from hydrogen, deuterium, halogen, hydroxy, nitro, cyano, methyl, ethyl, propyl, isopropyl, trifluoromethyl, methoxy, ethoxy, cyclopropyl;
R⁶ is independently selected from hydrogen, deuterium, halogen, hydroxy, nitro, cyano, methyl, ethyl, propyl, isopropyl, trifluoromethyl, methoxy, ethoxy, cyclopropyl;
R⁷ is independently selected from substituted phenyl, thienyl, 2-substituted thienyl, 2-pyridyl, 3-pyridyl, 4-pyridyl, pyrimidinyl, pyrrolyl, imidazolyl, pyrazolyl, oxazolyl, pyrazinyl, pyridazinyl, 2-furyl, 2-substituted furyl, quinolyl, isoquinolyl, naphthyl, indolyl, substituted indolyl;
The said substituents in the 2-substituted furyl, substituted phenyl and substituted indolyl are C1-C4 alkyl, C1-C4 haloalkyl, C1-C4 alkoxy and C1-C4 alkanol group, cyano, halogen, hydroxyl and amino .

As a preferred embodiment of the present invention, the above-mentioned 1-aminobenzo [4, 5] imidazo [1, 2-a] pyrazine-3-formamide compound is selected from any one of the following structural formulas:

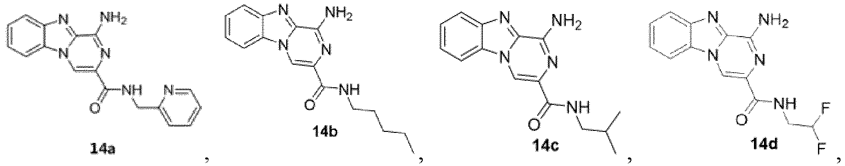

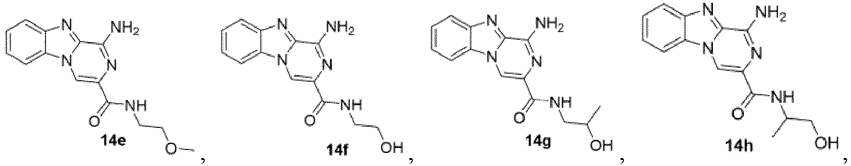

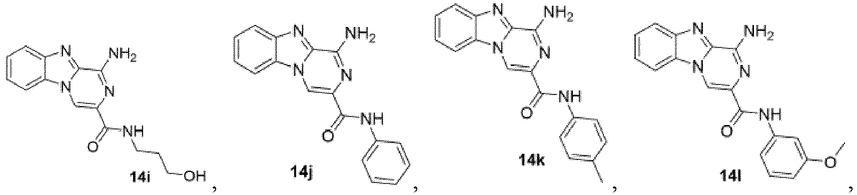

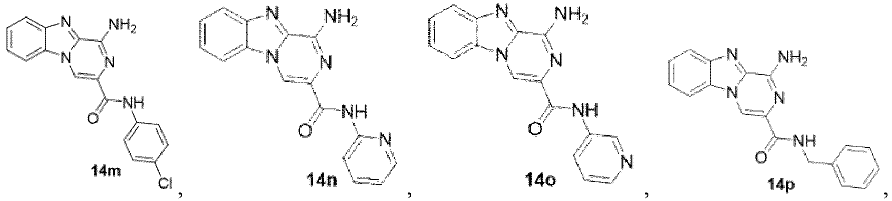

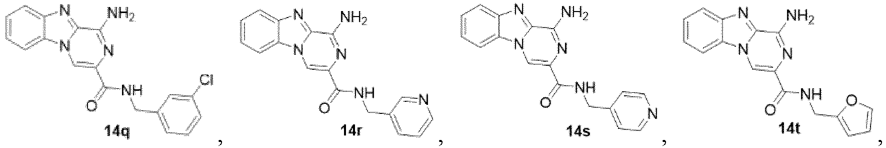

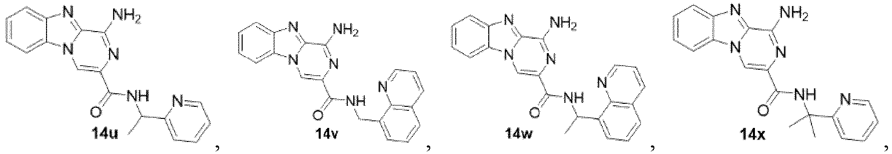

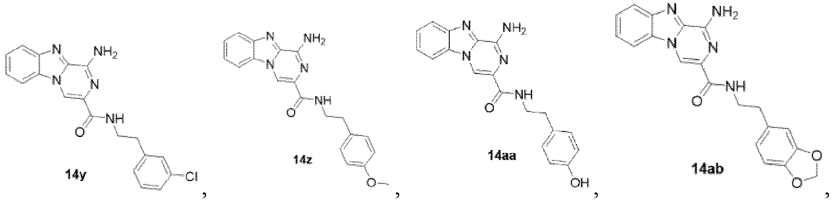

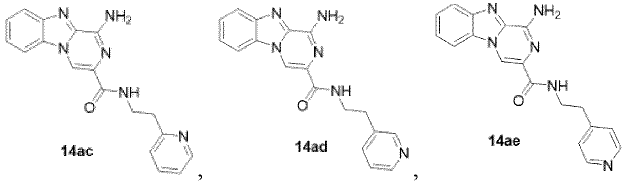

The said second purpose of the present invention is achieved by the following technical solution:
The method for preparing of the 1-aminobenzo [4, 5]imidazo [1, 2-a] pyrazine-3-formamide compound comprises the following steps:
S1, according to the following reaction formula. The compound shown in the Formula 1 was dissolved in an acidic solvent and cooled to 0-10 °C, and then the compound shown in the Formula 2 was added dropwise, and then transferred to room temperature for reaction, to obtain the compound shown in the Formula 3: 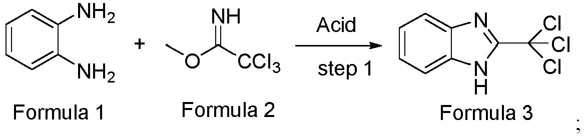
S2, according to the following reaction formula. The compound shpwn in the Formula 3 was cooled to 0-10 °C, and added the ammonia solution of 1, 4- dioxane. The reaction was carried out at room temperature under sealed condition to obtain the compound shown in the Formula 4: 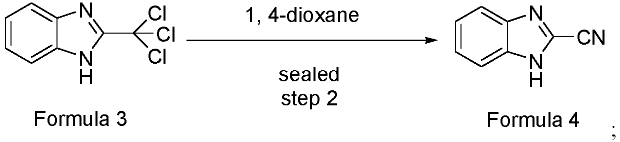
S3, according to the following reaction formula. The compound shown in the Formula 4 was dissolved in an alkaline organic solvent and cooled to -20-0 °C, and added the compound shown in the Formula 5, and then the reaction was carried out at this temperature to obtain the compound shown in the Formula 6: 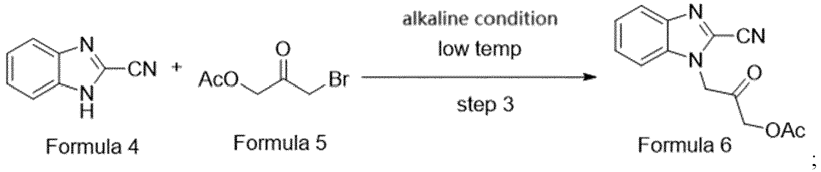
S4, according to the following reaction formula. The compound shown in the Formula 6 was mixed with ammonium salt and acid And the mixture was heated to 80-100 °C, and then the reaction was carried out at this temperature to obtain the compound shown in the Formula 7: 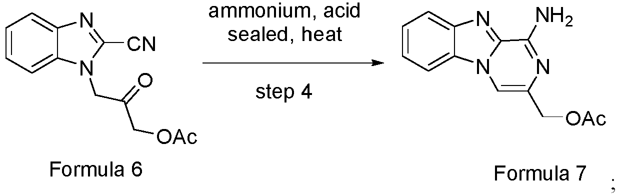
S5, according to the following reaction formula. The compound shown in the Formula 7 was dissolved in an alkaline organic solvent and the mixture was cooled to 0 °C, and added Di-tert-butyl dicarbonate, and then transferred to room temperature for reaction, to obtain the compound shown in the Formula 8: 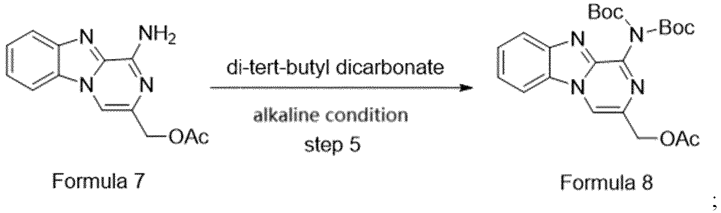
S6, according to the following reaction formula. The compound shown in the Formula 8 was dissolved in an organic solvent and cooled to 0 °C, and added the alkaline substance, and then transferred to room temperature for reaction to obtain the compound shown in the Formula 9: 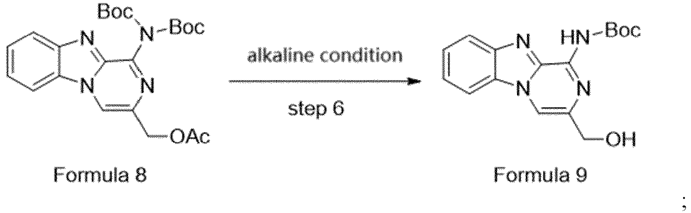
S7, according to the following reaction formula. The compound shown in Formula 9 was dissolved in organic solvent and cooled to 0 °C, and added the oxidizing agent, and then transferred to room temperature for reaction to obtain the compound shown in the Formula 10: 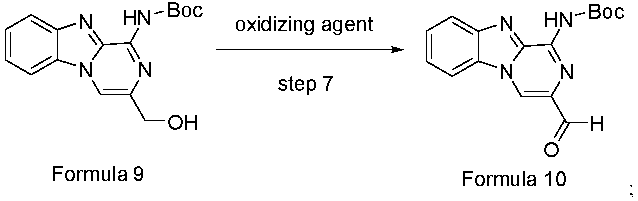
S8, according to the following reaction formula. The compound shown in the Formula 10 was dissolved in organic solvent and cooled to 0 °C and added the oxidizing agent, and then the reaction was carried out at this room temperature to obtain the compound shown in the Formula 11: 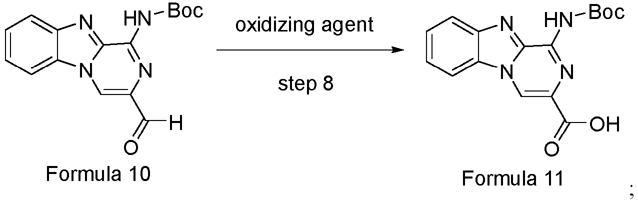
S9, according to the following reaction formula. The compounds shown in Formula 11 and Formula 12 were dissolved in organic solvent and cooled to 0 °C and added condensing agent, and then transferred to room temperature for reaction to obtain the compound shown in the Formula 13: 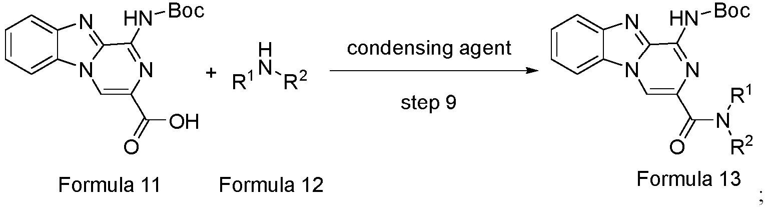
S10, according to the following reaction formula. The compound shown in the Formula 13 was dissolved in organic solvent and cooled to 0 °C, and added acidic substance, and.then transferred to room temperature for reaction to obtain the target compound:

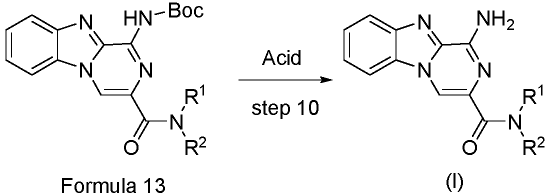

When the R¹ group in the compound shown in the formula I obtained by the preparation process needs to be modified. The modification can be performed by referring to a conventional group modification method. For example, when R² is 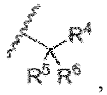 and when R⁴ is substituted phenyl, the substituent is 4-hydroxy, the compound in which R⁴ is 4-methoxyphenyl can be subjected to conventional reaction to prepare the compound in which R⁴ is 4-hydroxyphenyl.

The compound of this present invention has a novel structure and the chemical synthesis method is simple, the conditions are mild, the product yield and purity is high. It can be produced and prepared on a large industrial scale, which is convenient for promotion and application

Preferably, in step S1, the said acidic solvent includes acetic acid, propionic acid, and DMF (*N*, *N*-dimethylformamide). Specifically, the acidic solvent is acetic acid.

Preferably, in step S1, the molar mass ratio of the compound shown in the Formula 1 to the compound shown in the Formula 2 is 1:1.

Preferably, in step S2, the molar mass ratio of the compound shown in the Formula 3 to ammonia is 1: 3-4.

More preferably, in step S2, the molar mass ratio of the compound shown in the Formula 3 to ammonia is 1:4;

Preferably, in step S3, the said alkaline substance added to the basic organic solvent comprises triethylamine, *N, N*-diisopropylethylamine, potassium carbonate and cesium carbonate; And the said organic solvent comprises acetonitrile, dichloromethane, DMF (*N, N*-dimethylformamide), DMSO (dimethylsulfoxide), NMP (*N*-methylpyrrolidone). Specifically, the said alkaline substance is *N, N-*diisopropylethylamine (DIPEA), and the said organic solvent is acetonitrile.

Preferably, in step S3, the molar mass ratio of the compound shown in the Formula 4 to the compound of Formula 5 is 1:1.

Preferably, in the step S4, the said ammonium salt includes ammonium acetate, ammonium sulfate and ammonium nitrate. And the said acid comprises acetic acid and glacial acetic acid. Specifically, the said ammonium salt is ammonium acetate and the said acid is acetic acid.

Preferably, in step S4, the molar mass ratio of the compound shown in the Formula 6 to the ammonium salt is 1:6, and the reaction temperature is 95 °C.

Preferably, in step S5, the alkaline substance added to the alkaline organic solvent comprises triethylamine and *N, N*-diisopropylethylamine; The organic solvent comprises dichloromethane, tetrahydrofuran and 1, 2-dichloroethane. Specifically, the said alkaline substance is triethylamine, and the said organic solvent is tetrahydrofuran.

Preferably, in step S5, the molar mass ratio of the compound shown in the Formula 7 to di-tert-butyl dicarbonate is 1:2.

Preferably, in step S6, the said alkaline substance comprises potassium carbonate, cesium carbonate, lithium hydroxide, sodium hydroxide, and potassium hydroxide; The said organic solvent is methanol, ethanol and acetonitrile. Specifically, the said alkaline substance is potassium carbonate, and the said organic solvent is methanol.

Preferably, in step S6, the molar mass ratio of the compound shown in the Formula 8 to the alkaline substance is 1:2.

Preferably, in step S7, the said oxidizing agent comprises Dess-Martin periodinane and pyridinium chlorochromate. Specifically, the said oxidizing agent is Dess-Martin periodinane.

More preferably, in step S7, the molar mass ratio of the compound shown in the Formula 9 to the oxidizing agent is 1:1.

Preferably, in step S8, the said oxidizing agent is sodium hypochlorite, and the said organic solvent comprises tetrahydrofuran and 2-methyltetrahydrofuran. Specifically, the said organic solvent is tetrahydrofuran.

Preferably, in step S8, the molar mass ratio of the compound shown in the Formula 10 to the oxidizing agent is 1:2.

Preferably, in step S9, the compound shown in the Formula 12 is selected from 2-aminomethylpyridine, n-pentylamine, isobutylamine, 2,2-difluoroethylamine, 2-methoxyethylamine, ethanolamine, isopropanolamine, DL-aminopropanol, 3-amino-1-propanol, substitues 2-aminomethylpyridine, aniline, p-toluidine, m-aminoanisole, p-chlorobenzeneamine, 2-aminopyridine, 3-aminopyridine, benzylamine, 3-chlorobenzylamine, 3-aminomethylpyridine, 4-aminomethylpyridine, 2-furylmethylamine, 1- (2-)Pyridine) ethylamine substitues 2-aminomethylpyridine, (quinoline-8-methyl) amine, 1- (8-quinoline) ethylamine, 2- (pyridin-2-yl) -2-propylamine, 2- (3-chlorophenyl)ethylamine and 2- (4-methoxyphenyl) ethylamine.

Preferably, in step S9, the said condensing agent comprises a mixture of DMAP (4-dimethylaminopyridine) and DCC (dicyclohexylcarbodiimide), a mixture of DMAP and EDCI (1-(3-dimethylpyridine)),Aminopropyl) -3-ethylcarbodiimide hydrochloride), HATU (2- (7-azobenzotriazole) *-N, N, N ',N* '-tetramethyluronium hexafluorophosphate) and DIPEA (*N, N-*diisopropylethylamine), a mixture of HOBt (1-hydroxybenzotriazole) and DCC, a mixture of HOBt and EDCI. Specifically, the condensing agent is a mixture of HOBt and DCC, and the molar ratio of HOBt to DCC is 1:1.

Preferably, in step S9, the molar mass ratio of the compound shown in the Formula 11 to the compound shown in the Formula 12 is 1:1.

Preferably, in step S10, the said acidic substance comprises trifluoroacetic acid, formic acid, and concentrated hydrochloric acid. Specifically, the said acidic substance is trifluoroacetic acid.

Preferably, in step S10, the molar mass ratio of the compound shown in the Formula 13 to the acidic substance is 1: 10.

The said third purpose of the presented invention is achieved by the following technical solution:
The application of the 1-aminobenzo [4, 5] imidazo [1, 2-a] pyrazine-3-formamide compound in the preparation of adenosine receptor inhibitors or anti-tumor drugs.

Preferably, the said adenosine receptor is an adenosine A_{2A} receptor, and the anti-tumor drug is a tumor immunotherapy drug targeting the A_{2A} receptor.

The 1-aminobenzo [4, 5] imidazo [1, 2-a] pyrazine-3-formamide compound of the invention has good inhibition effect on adenosine A_{2A} receptor. Wherein, the compounds **14a** to **14e**, compound **14k,** compound **14o**, compound **14p**, compounds **14r** to **14u**, compound **14w**, compound **14x** and compounds **14ac** to **14ae**, inhibit adenosine A_{2A} receptor by more than 70%. Especially, the inhibitory rate of compounds **14a**, **14r**, **14s**, **14u** and **14ac** are more than 80%, and show excellent inhibition effect on adenosine A_{2A} receptors. These compounds can be prepared to be adenosine A_{2A} receptor inhibitors or tumor immunotherapy drugs targeting the adenosine A_{2A} receptor.

Specifically, the present invention provides an adenosine receptor inhibitor. The inhibitor uses the 1-aminobenzo [4, 5] imidazo [1, 2-a] pyrazine-3-formamide compound according to any one of claims 1-3, as the main active ingredient.

Specifically, the present invention also provides an anti-tumor drug. The drug uses the 1-aminobenzo [4, 5] imidazo [1, 2-a] pyrazine-3-formamide compound according to any one of claims 1-3 as the main active ingredient.

Compared with the prior art, the present invention has the following beneficial effects:
The present invention discloses a series of 1-aminobenzo [4, 5] imidazo [1, 2-a] pyrazine-3-formamide compounds with a novel structure and is an adenosine receptor inhibitor, has good inhibitory activity on adenosine A_{2A} receptors, has good subtype selectivity, and can be used as a targeted adenosine A_{2A} receptor inhibitor for immunotherapy. By means of inhibiting the activity of the adenosine A_{2A} receptor, the function of removing tumor cells by the immune system is released, so that the effect of treating tumors is achieved. Furthermore, the method for preparing the compound of the present invention is simple, uses cheap and easily available compounds as raw materials, has mild reaction conditions and high total yield and purity, can achieve dozens of grams of scale production, and supports the research of the clinical candidate adenosine A_{2A} receptor inhibitor in the later stage.

### Preferred embodiments of the invention

Specific solutions of the present invention are further illustrated below. It should be noted that the illustration of these embodiments is provided to assist in understanding the present invention, and does not constitute a limitation of the present invention. In addition, the technical features involved in the various embodiments of the present invention described below may be combined with each other as long as they do not conflict with each other.

The experimental methods in the following examples are all conventional methods unless otherwise specified, and the experimental materials used in the following examples, unless otherwise specified, can be purchased through conventional commercial channels.

### Example 1 Preparation of the 1-aminobenzo[4,5]imidazo[1,2-a]pyrazine-3-formamide compounds (Compound 14a)

The specific preparation process is as follows:

### (1) Preparation of the compound shown in Formula 3:

The chemical reaction formula of the preparation process is as follows:

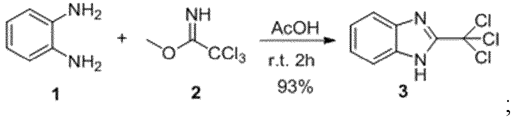

According to the above reaction formula, the compound shown in Formula 1 (1,2-phenylenediamine, 3.2 g, 30 mmol) and 50mL of acetic acid were added into a reaction flask. And the initial raw material, namely, the compound shown in Formula 1 (methyl 2,2,2-trichloroiminoacetate, 4 mL, 30 mmol) was slowly added after the reaction flask was cooled through ice bath, then stirred at room temperature for 2 hours. After TLC showed that the reaction was complete, the reactants were filtered, and the resulting filter cake was washed with water (3 times, 25 mL each time). Finally, the compound shown in Formula 3 (2-trichloromethyl-benzopyrimidine) (7.1 g, 93%) was obtained by vacuum drying.

### (2) Preparation of the compound shown in Formula 4:

The chemical reaction formula of the preparation process is as follows:

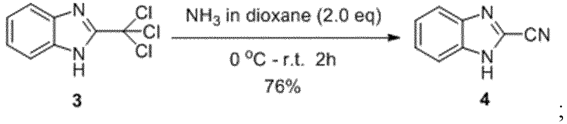

According to the above reaction formula, the compound shown in Formula 3 (2-trichloromethylbenzopyrimidine, 5.9 g, 25 mmol) was cooled to 0 °C. A solution of ammonia in 1,4-dioxane (0.40 M, 125 mL) was then added at 0 °C. The flask was sealed and the mixture was stirred at room temperature for 2 hours. After TLC showed that the reaction was complete, the mixture was concentrated under reduced pressure and subjected to column chromatography to afford the compound shown in Formula 4 (2-cyanobenzopyrimidine, 2.7 g, 76%).

### (3) Preparation of the compound shown in Formula 6:

The chemical reaction formula of the preparation process is as follows:

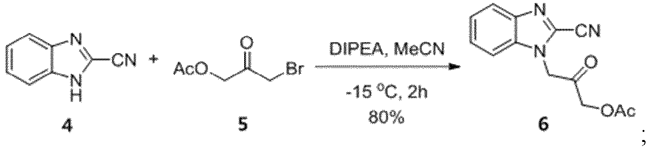

According to the above reaction formula, MeCN (40 mL), DIPEA (4.4 g, 34 mmol) and the compound shown in Formula 4 (2-cyanobenzopyrimidine, 2.6 g, 17 mmol) were added to a reaction flask and cooled to -15 °C. At this temperature, the compound shown in Formula 5 (3-bromo-2-oxopropyl acetate, 3.3 g, 17mmol) was added and the mixture was stirred at -15 °C for 3 hours. After TLC showed that the reaction was complete, the mixture was diluted with 150 mL of water, and extracted by ethyl acetate, dried with anhydrous sodium sulfate, concentrated under reduced pressure and subjected to column chromatography to afford the compound shown in Formula 6 (1-(2-(4-methoxyphenyl)-2-oxaethyl)-1H-benzopyrimidine-2-cyanide, 3.5 g, 80%). The characterization information of the product is as follows:
¹H NMR (400 MHz, Chloroform-*d*) δ 7.89-7.86 (m, 1H), 7.50-7.40 (m, 2H), 7.30-7.28 (m, 1H), 5.28 (s, 2H), 4.81 (s, 2H), 2.22 (s, 3H).

### (4) Preparation of the compound shown in Formula 7:

The chemical reaction formula of the preparation process is as follows:

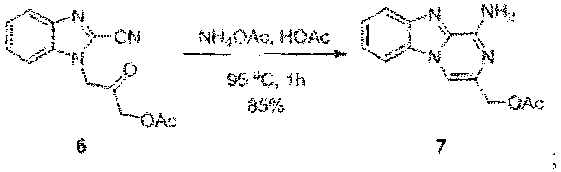

According to the above reaction formula, the compound shown in Formula 6 (3- (2-cyano-1H-benzo[d]imidazol-1-yl)-2-oxopropyl acetate), 3.3 g, 13 mmol), ammonium acetate (5.1 g, 66 mmol), and 5 mL of acetic acid were added to a sealed tube and stirred at 95 °C for 1 hour. After TLC showed that the reaction was complete, saturated sodium bicarbonate solution was added to neutralize the reaction until no bubbles were generated. The mixture was then extracted with dichloromethane (3 times, 100 mL of dichloromethane each time). The organic phases were collected, dried with anhydrous sodium sulfate, concentrated under reduced pressure and subjected to column chromatography to afford the compound shown in Formula 7 (methyl (1-aminobenzo[4,5]imidazo[1,2-a]pyrazin-3-yl)acetate, 2.8 g, 85%). The characterization information of the product is as follows:
¹H NMR (500 MHz, Chloroform-*d*) δ 7.96-7.86 (m, 3H), 7.56-7.46 (m, 3H), 6.06 (s, 2H), 5.10 (s, 2H), 2.16 (s, 3H).

### (5) Preparation of the compound shown in Formula 8:

The chemical reaction formula of the preparation process is as follows:

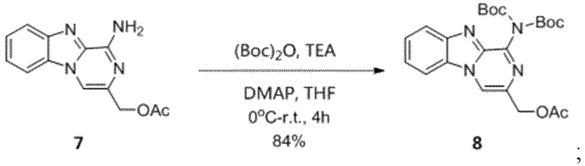

According to the above reaction formula, the compound shown in Formula 7 (methyl (1-aminobenzo[4,5]imidazo[1,2-a]pyrazin-3-yl)acetate, 2.8 g, 11 mmol), DMAP (134 mg, 1.1 mmol), triethylamine (4.5 g, 44 mmol) were dissolved in 50 mL of tetrahydrofuran and cooled to 0 °C, di-tert-butyl dicarbonate (6.0 g, 27.5 mmol) was then slowly added dropwise at 0 °C and stirred at room temperature for 4 hours. After TLC showed that the reaction was complete, 150 mL of water was added. The mixture was extracted with dichloromethane (3 times, 100 mL of dichloromethane each time), and the organic phases were combined, washed with 100 mL of saturated saline and dried with sodium sulfate, concentrated under reduced pressure and subjected to column chromatography to afford the compound shown in Formula 8 (methyl (1-(di(t-butoxycarbonyl)amino)benzo[4,5]imidazo[1,2-a]pyrazin-3-yl)acetate, 4.2 g, 84%). The characterization information of the product is as follows:
¹H NMR (400 MHz, Chloroform-*d*) 8.46 (s, 1 H), 8.07 (d, *J*=8.3 Hz, 1 H), 7.97 (d, *J*=8.3 Hz, 1 H), 7.64 (t, *J*=7.7 Hz, 1 H), 7.53 (t, *J*=7.7 Hz, 1 H), 5.29 (s, 2 H), 2.16 (3 H, s), 1.41 (s, 18 H).

### (6) Preparation of the compound shown in Formula 9:

The chemical reaction formula of the preparation process is as follows:

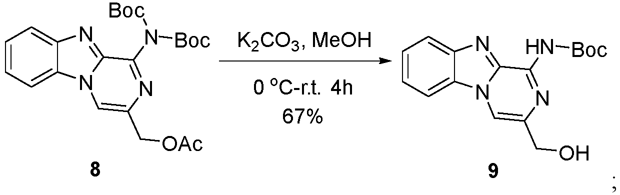

According to the above reaction formula, compound shown in Formula 8 (Methyl (1-(di(t-butoxycarbonyl)amino)benzo[4,5]imidazo[1,2-a]pyrazin-3-yl)acetate, 4.1 g, 9 mmol) were dissolved in 50 mL of methanol and cooled to 0 °C. Potassium carbonate (3.7 g, 27 mmol) was slowly added at 0 °C and the mixture was stirred at room temperature for 4 hours. After TLC showed that the reaction was complete, 200 mL of water was added, and the mixture was extracted with dichloromethane (3 times, 100 mL of dichloromethane each time), and the organic phases were combined, washed with saturated saline (100 mL), dried with anhydrous sodium sulfate, concentrated under reduced pressure, and subjected to column chromatography to afford the compound shown in Formula 9 (tert-butyl (3-(hydroxymethyl)benzo[4,5]imidazo[1,2-a]pyrazin-1-yl)carbamate, 67%, 1.9 g). The characterization information of the product is as follows:
¹H NMR (400 MHz, Chloroform-*d*) δ 8.56 (s, 1H), 8.12 (s, 1H), 7.96 (d, *J*=8.3 Hz, 1H), 7.89 (d, *J*=8.3 Hz, 1H), 7.61-7.57 (m, 1H), 7.50-7.60 (m, 1H), 4.85 (s, 2H), 1.56 (s, 9H).

### (7) Production of the compound shown in Formula 10:

The chemical reaction formula of the preparation process is as follows:

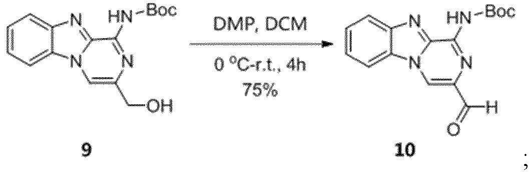

According to the above reaction formula, the compound shown in Formula 9 (tert-butyl (3-(hydroxymethyl)benzo[4,5]imidazo[1,2-a]pyrazin-1-yl)carbamate, 1.9 g, 6 mmol) were dissolved in 50 mL of dichloromethane and cooled to 0 °C, then Dess-Martin periodinane (3.6 g, 8.4 mmol) was added slowly at 0 °C and the mixture was stirred at room temperature for 4 hours. The mixture was filtered after TLC showed that the reaction was completed. Then saturated sodium bicarbonate solution was added until no bubbles were generated. The mixture was extracted with dichloromethane (3 times, 100 mL of dichloromethane each time) and the organic phases were combined, washed with saturated saline (100 mL), dried with anhydrous sodium sulfate, concentrated under reduced pressure, and subjected to column chromatography to afford the compound shown in Formula 10 (tert-butyl (3-formylbenzo[4,5]imidazo[1,2-a]pyrazin-1-yl)carbamate, 1.5 g, 81%). The characterization information of the product is as follows:
¹H NMR (500 MHz, Chloroform-*d*) δ 10.17 (s, 1H), 8.82 (s, 1H), 8.03 - 8.00 (m, 2H), 7.70-7.67 (m, 1H), 7.61-7.57 (m, 1H), 1.61 (s, 9H).

### (8) Preparation of the compound shown in Formula 11:

The chemical reaction formula of the preparation process is as follows:

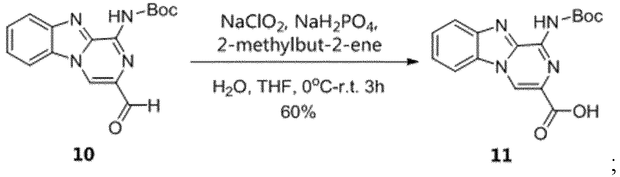

According to the above reaction formula, compound shown in Formula 10 (tert-butyl (3-formylbenzo[4,5]imidazo[1,2-a]pyrazin-1-yl)carbamate, 1.4 g, 4.5 mmol), sodium dihydrogen phosphate (2.2 g, 18 mmol), isopentene (1.6 g, 22 mmol) were dissolved in a mixed solution of 40 mL of tetrahydrofuran and 20 mL of water, and the mixture was cooled to 0 °C. Sodium chlorite (1.6 g, 18 mmol) was added slowly at 0 °C and the mixture was stirred at 0 °C for 4 hours. After TLC showed that the reaction was completed, sodium thiosulfate (3.6 g, 22 mmol) aqueous solution was added at 0 °C to quench the reaction. The mixture was further extracted with dichloromethane (3 times, 100 mL of dichloromethane each time), and the organic phases were combined, washed with saturated saline (100 mL), dried with anhydrous sodium sulfate and concentrated under reduced pressure. The obtained solid was washed with n-hexane to afford the compound shown in Formula 11 (1-((tert-butoxycarbonyl)amino)benzo[4,5]imidazo[1,2-a]pyrazine-3-carboxylic acid, 886 mg, 60%). The characterization information of the product is as follows:
¹H NMR (400 MHz, DMSO-*d*₆) δ9.46 (s, 1H), 8.56 (s, 1H), 7.98-7.96 (m, 1H), 7.65-7.54 (m, 2H), 1.50 (s, 9H).

### (9) Preparation of the compound shown in Formula 13:

The chemical reaction formula of the preparation process is as follows:

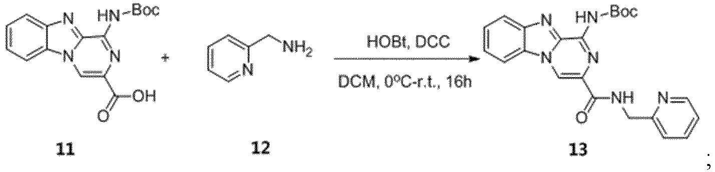

According to the above reaction formula, the compound shown in Formula 11 (1-((t-butoxycarbonyl)amino)benzo[4,5]imidazo[1,2-a]pyrazine-3-carboxylic acid), 67 mg, 0.2 mmol), the compound shown in Formula 12 (2-aminomethylpyridine, 32 mg, 0.3 mmol), HOBt (41 mg,0.3 mmol) were dissolved in 2 mL of dichloromethane and cooled to 0 °C, then DCC (62 mg, 0.3 mmol) was added slowly at 0 °C. The mixture was stirred at room temperature for 16 hours. After TLC showed that the reaction was complete, the mixture was filtered on silica gel and washed with a mixture of dichloromethane/methanol (dichloromethane: methanol = 50:1). The filtrate was concentrated under reduced pressure to afford a crude product of the compound shown in Formula 13 (tert-butyl (3-((pyridin-2-ylmethyl)carbamoyl)benzo[4,5]imidazo[1,2-a]pyrazin-1-yl)carbamate). The characterization information of the product is as follows:
¹H NMR (400 MHz, Chloroform-*d*) δ 9.00 (s, 1H), 8.95 - 8.92 (m, 1H), 8.63 (d, *J =* 4.8 Hz, 1H), 8.49 (s, 1H), 8.00 (d, *J =* 8.5 Hz, 2H), 7.70 - 7.62 (m, 2H), 7.58 - 7.54 (m, 1H), 7.39 (d, *J =* 7.8 Hz, 1H), 7.22 - 7.19 (m, 1H), 4.85 (d, *J =* 5.8 Hz, 2H), 1.60 (s, 9H).

### (10) Preparation of Target Compound:

The chemical reaction formula of the preparation process is as follows:

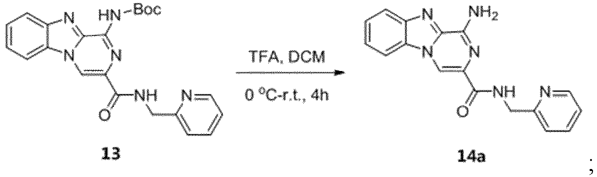

The crude product of the compound shown in Formula 13 (tert-butyl (3-((pyridin-2-ylmethyl)carbamoyl)benzo[4,5]imidazo[1,2-a]pyrazin-1-yl)carbamate) was dissolved in 2 mL of dichloromethane and cooled to 0 °C. Then 0.2 mL of trifluoroacetic acid was slowly added at 0 °C, and stirred at room temperature for 4 hours. After TLC showed that the reaction was complete, the saturated sodium bicarbonate aqueous solution was added until no bubbles were generated. The mixture was extracted with dichloromethane (3 times, 100 mL of dichloromethane each time), and the organic phases were combined, washed with saturated saline (50 mL), dried over anhydrous sodium sulfate, concentrated under reduced pressure, and subjected to column chromatography to afford the target compound **14a** (1-amino-*N*-(pyridin-2-ylmethyl)benzo[4,5]imidazo[1,2-a]pyrazine-3-formamide, 45 mg, 70% (this yield is the yield of steps (9) and (10). The characterization information of the product is as follows:
¹H NMR (500 MHz, DMSO-*d*₆)δ8.95 (s, 1H), 8.81 (t, *J=* 6.0 Hz, 1H), 8.56 (d, *J=* 4.8 Hz, 1H), 8.45 (d, *J=* 8.2 Hz, 1H), 7.92 (d, *J=* 8.2 Hz, 1H), 7.78 (t, *J=* 7.7 Hz, 1H), 7.56-7.46 (m, 4H), 7.39 (d, *J=* 7.8 Hz, 1H), 7.30 (t, *J=* 6.2 Hz, 1H), 4.68 (d, *J=* 5.8 Hz, 2H). ¹³C NMR (126 MHz, DMSO-*d*₆) δ164.2, 158.2, 149.4, 143.6, 137.3, 136.3, 130.6, 130.2, 126.4, 123.6, 122.8, 121.9, 120.7, 113.6, 111.5, 44.6.

### Example 2 Preparation of the 1-aminobenzo[4,5]imidazo[1,2-a]pyrazine-3-formamide compounds (Compound 14b)

The preparation process of the compound **14b** was the same as the preparation process of compound **14a** in Example 1, except that: in step S9, the compound shown in Formula 13 was prepared by using n-pentylamine instead of 2-aminomethylpyridine to finally afford the compound 14b (46 mg, 70%). The chemical structural formula is as follows:

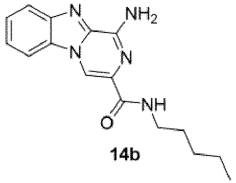

The characterization information of the product is as follows:
¹H NMR (500 MHz, Chloroform-*d*) δ 8.75 (s, 1H), 7.94 (dd, *J=* 17.1, 8.3 Hz, 2H), 7.77 (t, *J=* 5.3 Hz, 1H), 7.59-7.56 (m, 1H), 7.51-7.47 (m, 1H), 5.88 (s, 2H), 3.48 (q, *J=* 6.8 Hz, 2H), 1.64 (p, *J*= 7.3 Hz, 2H), 1.43-1.38 (m, 2H), 1.36-1.32 (m, 3H), 0.91-0.89 (m, 3H). ¹³C NMR (126 MHz, Chloroform-*d*) δ 163.7, 148.1, 143.7, 135.7, 130.1, 129.7, 126.6, 123.8, 121.0, 111.7, 39.5, 31.5, 29.7, 26.7, 22.6, 14.0.

### Example 3 Preparation of the 1-aminobenzo[4,5]imidazo[1,2-a]pyrazine-3-formamide compounds (Compound 14c)

The preparation process of compound **14c** was the same as the preparation process of compound **14a** in Example 1, except that: in step S9, the compound shown in Formula 13 was prepared by using isobutylamine instead of 2-aminomethylpyridine to finally afford the compound **14c** (43 mg, 70%). The chemical structural formula is as follows:

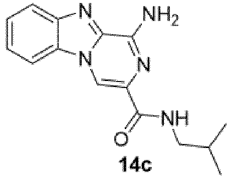

The characterization information of the product is as follows:
¹H NMR (500 MHz, Chloroform-*d*) δ 8.76 (s, 1H), 7.96 (dd, *J=* 16.6, 8.3 Hz, 2H), 7.84 (s, 1H), 7.59 (t, *J=* 7.7 Hz, 1H), 7.51 (t, *J=* 7.7 Hz, 1H), 5.77 (s, 2H), 3.33 (t, *J=* 6.6 Hz, 2H), 1.02 (d, *J*= 6.7 Hz, 6H). ¹³C NMR (126 MHz, DMSO-*d*₆) δ 163.9, 149.3, 143.6, 136.2, 130.6, 130.3, 126.4, 123.5, 120.6, 113.6, 111.2, 46.5, 28.8, 20.5.

### Example 4 Preparation of the 1-aminobenzo[4,5]imidazo[1,2-a]pyrazine-3-formamide compounds (Compound 14d)

The preparation process of compound **14d** was the same as the preparation process of compound **14a** in Example 1, except that: in step S9, the compound shown in Formula 13 was prepared by using 2,2-difluoroethylamine instead of 2-aminomethylpyridine to finallly afford the compound 3 (44 mg, 75%). The chemical structural formula is as follows:

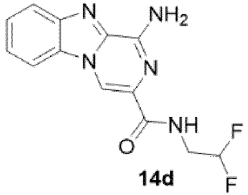

The characterization information of the product is as follows:
¹H NMR (500 MHz, DMSO-*d*₆) δ 8.97 (s, 1H), 8.46-8.44 (m, 1H), 8.41 (t, *J=* 6.2 Hz, 1H), 7.92 (d, *J=* 8.2 Hz, 1H), 7.58-7.55 (m, 1H), 7.52-7.47 (m, 3H), 6.32-6.01 (m, 1H), 3.80 (tt, *J=* 15.8, 4.2 Hz, 2H). ¹⁹F NMR (471 MHz, DMSO-*d*₆) δ-122.25.

### Example 5 Preparation of the 1-aminobenzo[4,5]imidazo[1,2-a]pyrazine-3-formamide compounds (Compound 14e)

The preparation process of compound **14e** was the same as the preparation process of compound **14a** in Example 1, except that: in step S9, the compound shown in Formula 13 was prepared by using 2-methoxyethylamine instead of 2-aminomethylpyridine to finally afford the compound **14e** (40 mg, 71%). The chemical structural formula is as follows:

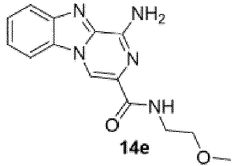

The characterization information of the product is as follows:
¹H NMR (500 MHz, Chloroform-*d*) δ 8.75 (s, 1H), 8.06 (s, 1H), 7.96 (dd, *J=* 17.6, 8.2 Hz, 2H), 7.59 (t, *J=* 7.5 Hz, 1H), 7.51 (t, *J=* 7.6 Hz, 1H), 5.79 (s, 2H), 3.69 (q, *J=* 5.2 Hz, 2H), 3.61 (t, *J*= 4.9 Hz, 2H), 3.43 (s, 3H). ¹³C NMR (126 MHz, DMSO-*d*₆) δ 164.0, 149.4, 143.6, 136.2, 130.6, 130.1, 126.4, 123.5, 120.6, 113.6, 111.3, 71.1, 58.4, 38.9.

### Example 6 Preparation of the 1-aminobenzo[4,5]imidazo[1,2-a]pyrazine-3-formamide compounds (Compound 14f)

The preparation process of compound **14f** was the same as the preparation process of compound **14a** in Example 1, except that: in step S9, the compound shown in Formula 13 was prepared by using ethanolamine instead of 2-aminomethylpyridine to finally afford the compound 5 (27 mg, 50%). The chemical structural formula is as follows:

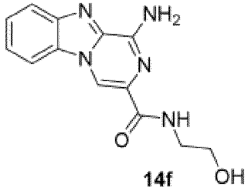

The characterization information of the product is as follows:
¹H NMR (500 MHz, DMSO-*d*₆) δ 8.90 (s, 1H), 8.43 (d, *J=* 8.2 Hz, 1H), 8.23 (t, *J=* 5.6 Hz, 1H), 7.92 (d, *J=* 8.2 Hz, 1H), 7.57-7.46 (m, 4H), 4.92 (t, *J=* 4.7 Hz, 1H), 3.56 (q, *J=* 4.8 Hz, 2H). 3.14 (t, *J=* 3.5 Hz, 2H). ¹³C NMR (126 MHz, DMSO-*d*₆) δ 164.0, 149.4, 143.6, 136.2, 130.6, 130.2, 126.4, 123.6, 120.6, 113.6, 111.2, 60.3, 41.9.

### Example 7 Preparation of the 1-aminobenzo[4,5]imidazo[1,2-a]pyrazine-3-formamide compounds (Compound 14g)

The preparation process of compound **14g** was the same as the preparation process of compound **14a** in Example 1, except tha:t in step S9, the compound shown in Formula 13 was prepared by using isopropanolamine instead of 2-aminomethylpyridine to finally afford the compound **14g** (30 mg, 53%). The chemical structural formula is as follows:

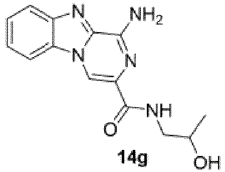

The characterization information of the product is as follows:
¹H NMR (400 MHz, DMSO-*d*₆) δ 8.91 (s, 1H), 8.44 (d, *J=* 8.1 Hz, 1H), 8.20-8.15 (m, 1H), 7.92 (d, *J=* 8.1 Hz, 1H), 7.58-7.55 (d, *J=* 12.1 Hz, 3H), 7.48 (t, *J=* 7.5 Hz, 1H), 4.94 (d, *J=* 4.2 Hz, 1H), 3.84-3.75 (m, 1H), 3.21-3.15 (M, 1H), 1.10 (d, *J=* 6.1 Hz, 3H). ³C NMR (126 MHz, DMSO-*d*₆) δ 163.9, 149.4, 143.5, 136.2, 130.6, 130.2, 126.4, 123.5, 120.6, 113.6, 111.2, 65.6, 46.6, 21.6.

### Example 8 Preparation of the 1-aminobenzo[4,5]imidazo[1,2-a]pyrazine-3-formamide compounds (compound 14h)

The preparation process of compound **14h** was the same as the preparation process of compound **14a** in Example 1, except that: in step S9, the compound shown in Formula 13 was prepared by using DL-aminopropanol instead of 2-aminomethylpyridine to finally afford the compound **14h** (28 mg,51%). The chemical structural formula is as follows:

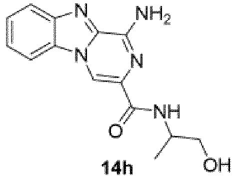

The characterization information of the product is as follows:
¹H NMR (500 MHz, DMSO-*d*₆) δ 8.89 (s, 1H), 8.43 (d, *J=* 8.2 Hz, 1H), 8.01 (d, *J=* 8.5 Hz, 1H), 7.92 (d, *J=* 8.2 Hz, 1H), 7.58-7.54 (m, 3H), 7.48 (t, *J=* 7.7 Hz, 1H), 4.97 (s, 1H), 4.07-4.04 (M, 1H), 3.50-3.47 (m, 2H), 1.18 (d, *J=* 6.6 Hz, 3H). ¹³C NMR (126 MHz, DMSO-*d*₆) δ 163.3, 149.4, 143.6, 136.2, 130.6, 130.3, 126.4, 123.5, 120.6, 113.6, 111.2, 64.6, 46.8, 17.9.

### Example 9 Preparation of the 1-aminobenzo[4,5]imidazo[1,2-a]pyrazine-3-formamide compounds (Compound 14i)

The preparation process of compound **14i** was the same as the preparation process of compound **14a** in Example 1, except that: in step S9, the compound shown in Formula 13 was prepared by using 3-amino-1-propanol instead of 2-aminomethylpyridine to finally afford the compound **14i** (32 mg,55%). The chemical structural formula is as follows:

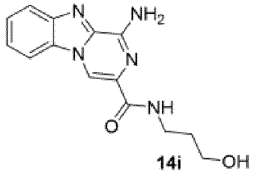

The characterization information of the product is as follows:
¹H NMR (500 MHz, DMSO-*d*₆) δ 8.90 (d, *J=* 1.6 Hz, 1H), 8.44 (d, *J=* 8.3 Hz, 1H), 8.22 (t, *J=* 6.0 Hz, 1H), 7.92 (d, *J=* 8.2 Hz, 1H), 7.56 (t, *J =* 6.8 Hz, 1H), 7.49-7.46 (m, 3H), 4.58 (t, *J=* 5.0 Hz, 1H), 3.51 (q, *J=* 5.3 Hz, 2H), 3.42 (q, *J=* 6.2, 5.6 Hz, 2H), 1.71 (p, *J=* 6.6 Hz, 2H). ¹³C NMR (126 MHz, DMSO-*d*₆) δ 164.0, 149.3, 143.6, 136.2, 130.6, 130.4, 126.4, 123.5, 120.6, 113.6, 111.2, 59.1, 36.6, 32.9.

### Example 10 Preparation of the 1-aminobenzo[4,5]imidazo[1,2-a]pyrazine-3-formamide compounds (Compound 14j)

The preparation process of compound **14j** was the same as the preparation process of compound **14a** in Example 1, except that: in step S9, the compound shown in Formula 13 was prepared by using aniline instead of 2-aminomethylpyridine to finally afford the compound **14j** (47 mg, 78%). The chemical structural formula is as follows:

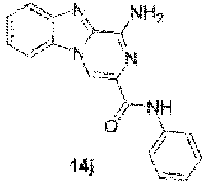

The characterization information of the product is as follows:
¹H NMR (500 MHz, DMSO-*d*₆) δ 10.09 (s, 1H), 9.08 (s, 1H), 8.49 (d, *J=* 8.2 Hz, 1H), 7.95 (d, *J=* 8.2 Hz, 1H), 7.81 (d, *J=* 7.9 Hz, 2H), 7.62-7.57 (m, 3H), 7.51 (t, *J=* 7.6 Hz, 1H), 7.41 (t, *J=* 7.5 Hz, 2H), 7.15 (t, *J*= 7.3 Hz, 1H). ¹³C NMR (126 MHz, DMSO-*d*₆) δ 162.5, 149.3, 143.7, 138.6, 136.2, 130.6, 130.0, 129.5, 126.5, 124.3, 123.7, 120.7, 120.0, 113.7, 112.1.

### Example 11 Preparation of the 1-aminobenzo[4,5]imidazo[1,2-a]pyrazine-3-formamide compounds (Compound 14k)

The preparation process of compound **14k** was the same as the preparation process of compound **14a** in Example 1, except that: in step S9, the compound shown in Formula 13 was prepared by using p-toluidine instead of 2-aminomethylpyridine to finally afford compound **14k** (48 mg,76%). The chemical structural formula is as follows:

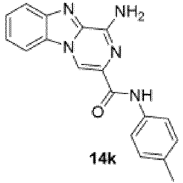

The characterization information of the product is as follows:
¹H NMR (400 MHz, DMSO-*d*₆) δ 9.99 (s, 1H), 9.05 (s, 1H), 8.49-8.47 (m, 1H), 7.94 (d, *J=* 7.9 Hz, 1H), 7.68-7.50 (m, 6H), 7.22-7.18 (d, *J=* 7.6 Hz, 2H), 2.28 (s, 3H). ¹³C NMR (101 MHz, DMSO-*d*₆) δ 162.3, 149.3, 143.7, 136.2, 136.1, 133.3, 130.6, 130.1, 129.8, 126.5, 123.7, 120.7, 120.0, 113.7, 112.0, 21.0.

### Example 12 Preparation of the 1-aminobenzo[4,5]imidazo[1,2-a]pyrazine-3-formamide compounds (Compound 14l)

The preparation process of compound 11 was the same as the preparation process of compound **14a** in Example 1, except that: in step S9, the compound shown in Formula 13 was prepared by using m-aminoanisole instead of 2-aminomethylpyridine to finally afford the compound 11 (46 mg, 69%). The chemical structural formula is as follows:

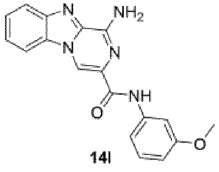

The characterization information of the product is as follows:
¹H NMR (500 MHz, DMSO-*d*₆) δ 10.06 (s, 1H), 9.07 (s, 1H), 8.48 (d, *J=* 8.0 Hz, 1H), 7.94 (d, *J=* 8.3 Hz, 1H), 7.61-7.51 (m, 6H), 7.36-7.31 (m, 2H), 6.72 (d, *J=* 8.1 Hz, 1H), 3.79 (s, 3H). ¹³C NMR (126 MHz, DMSO-*d*₆) δ 162.5, 160.2, 149.3, 143.7, 139.8, 136.2, 130.6, 130.3, 130.0, 126.6, 123.7, 120.7, 113.7, 112.3, 112.2, 109.8, 105.8, 55.55.

### Example 13 Preparation of the 1-aminobenzo[4,5]imidazo[1,2-a]pyrazine-3-formamide compounds (Compound 14m)

The preparation process of compound **14m** was the same as the preparation process of compound **14a** in Example 1, except that: in step S9, the compound shown in Formula 13 was prepared by using p-chloroaniline instead of 2-aminomethylpyridine to afford the compound **14m** (41 mg, 61%). The chemical structural formula is as follows:

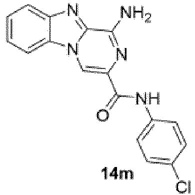

The characterization information of the product is as follows:
¹H NMR (500 MHz, DMSO-*d*₆) δ 10.21 (s, 1H), 9.08 (s, 1H), 8.49 (d, *J=* 7.0 Hz, 1H), 7.95-7.85 (m, 3H), 7.60-7.45 (m, 6H). ¹³C NMR (126 MHz, DMSO-*d*₆) δ 162.7, 149.3, 143.7, 137.7, 136.2, 130.6, 129.9, 129.3, 127.9, 126.6, 123.7, 121.7, 120.7, 113.7, 112.3.

### Example 14 Preparation of the 1-aminobenzo[4,5]imidazo[1,2-a]pyrazine-3-formamide compounds (Compound 14n)

The preparation process of compound **14n** was the same as the preparation process of compound **14a** in Example 1, except that: in step S9, the compound shown in Formula 13 was prepared by using 2-aminopyridine instead of 2-aminomethylpyridine to finally afford the compound **14n** (43 mg, 70%). The chemical structural formula is as follows:

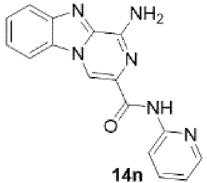

The characterization information of the product is as follows:
¹H NMR (500 MHz, DMSO-*d*₆) δ 10.33 (s, 1H), 9.14 (s, 1H), 8.51 (d, *J=* 8.1 Hz, 1H), 8.39 (s, 1H), 8.33 (d, *J=* 8.3 Hz, 1H), 7.96-7.91 (m, 2H), 7.81 (s, 2H), 7.59 (t, *J=* 7.7 Hz, 1H), 7.51 (t, *J=* 7.8 Hz, 1H), 7.20 (s, 1H). ¹³C NMR (126 MHz, DMSO-*d*₆) δ 162.5, 151.3, 149.5, 148.9, 143.7, 139.1, 136.3, 130.7, 129.1, 126.6, 123.8, 120.7, 120.4, 113.7, 113.3, 112.5.

### Example 15 Preparation of the 1-aminobenzo[4,5]imidazo[1,2-a]pyrazine-3-formamide compounds (Compound 14o)

The preparation process of compound **14o** was the same as the prerparation process of compound **14a** in Example 1, except that: in step S9, the compound shown in Formula 13 was prepared by using 3-aminopyridine instead of 2-aminomethylpyridine to finally afford the compound **14o** (35 mg, 59%). The chemical structural formula is as follows:

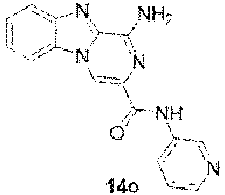

The characterization information of the product is as follows:
¹H NMR (500 MHz, DMSO-*d*₆) δ 10.29 (s, 1H), 9.11 (s, 1H), 8.99 (s, 1H), 8.49 (d, *J=* 8.1 Hz, 1H), 8.35-8.27 (m, 2H), 7.95 (d, *J=* 8.2 Hz, 1H), 7.60-7.43 (m, 5H). ¹³C NMR (126 MHz, DMSO-*d*₆) δ 163.3, 149.4, 145.2, 143.7, 142.1, 136.2, 135.5, 130.6, 129.8, 127.3, 126.6, 124.2, 123.7, 120.7, 113.7, 112.6.

### Example 16 Preparation of the 1-aminobenzo[4,5]imidazo[1,2-a]pyrazine-3-formamide compounds (Compound 14p)

The preparation process of compound **14p** was the same as the prepraration process of compound **14a** in Example 1, except that: in step S9, the compound shown in Formula 13 was prepared by using benzylamine instead of 2-aminomethylpyridine to finally afford the compound **14p** (46 mg, 73%). The chemical structural formula is as follows:

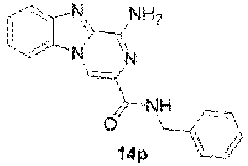

The characterization information of the product is as follows:
¹H NMR (500 MHz, DMSO-*d*₆) δ 8.94 (s, 1H), 8.60 (s, 1H), 8.44 (d, *J=* 7.3 Hz, 1H), 7.92 (d, *J=* 7.6 Hz, 1H), 7.59-7.52 (m, 1H), 7.47 (s, 3H), 7.36 (s, 4H), 7.27 (s, 1H), 4.56 (d, *J=* 5.1 Hz, 2H). ¹³C NMR (126 MHz, DMSO-*d*₆) δ 164.1, 149.4, 143.6, 139.8, 136.2, 130.6, 130.2, 128.9, 128.0, 127.5, 126.4, 123.5, 120.6, 113.6, 111.5, 42.8.

### Example 17 Preparation of the 1-aminobenzo[4,5]imidazo[1,2-a]pyrazine-3-formamide compounds (Compound 14q)

The preparation process of compound **14q** was the same as the prerparation process of compound **14a** in Example 1, except that: in step S9, the compound shown in Formula 13 was prepared by using 3-chlorobenzylamine instead of 2-aminomethylpyridine to finally afford the compound **14q** (44 mg, 62%). The chemical structural formula is as follows:

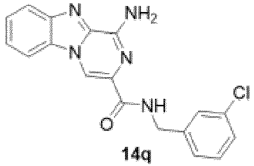

The characterization information of the product is as follows:
¹H NMR (500 MHz, DMSO-*d*₆) δ 8.94 (s, 1H), 8.77 (t, *J=* 6.5 Hz, 1H), 8.43 (d, *J=* 8.2 Hz, 1H), 7.92 (d, *J=* 8.2 Hz, 1H), 7.57 (t, *J=* 7.7 Hz, 1H), 7.50-7.45 (m, 3H), 7.41-7.37 (m, 2H), 7.33-7.32 (m, 2H), 4.55 (d, *J*= 6.3 Hz, 2H). ¹³C NMR (126 MHz, DMSO-*d*₆) δ 164.3, 149.4, 143.6, 142.6, 136.2, 133.5, 130.8, 130.6, 130.1, 127.7, 127.3, 126.6, 126.5, 123.6, 120.7, 113.6, 111.6, 42.3.

### Example 18 Preparation of the 1-aminobenzo[4,5]imidazo[1,2-a]pyrazine-3-formamide compounds (Compound 14r)

The preparation process of compound **14r** was the same as the preparation of compound **14a** in Example 1, except that: in step S9, the compound shown in Formula 13 was prepared by using 3-aminomethylpyridine instead of 2-aminomethylpyridine to finally afford the compound **14r** (50 mg,79%). The chemical structural formula is as follows:

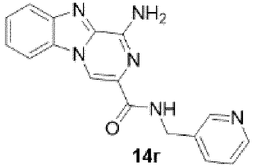

The characterization information of the product is as follows:
¹H NMR (500 MHz, DMSO-*d*₆) δ 8.96 (s, 1H), 8.74 (t, *J=* 5.9 Hz, 1H), 8.60 (s, 1H), 8.48 (d, *J=* 4.2 Hz, 1H), 8.45 (d, *J=* 8.2 Hz, 1H), 7.93 (d, *J=* 8.2 Hz, 1H), 7.77 (d, *J=* 7.6 Hz, 1H), 7.57 (t, *J*= 7.6 Hz, 1H), 7.52-7.42 (m, 3H), 7.41-7.35 (m, 1H), 4.59 (d, *J=* 6.1 Hz, 2H). ¹³C NMR (126 MHz, DMSO-*d*₆) δ 164.4, 149.5, 149.4, 148.7, 143.6, 136.2, 135.8, 135.4, 130.6, 130.2, 126.4, 124.0, 123.6, 120.6, 113.6, 111.6, 40.5.

### Example 19 Preparation of the 1-aminobenzo[4,5]imidazo[1,2-a]pyrazine-3-formamide compounds (Compound 14s)

The preparation process of compound **14s** was the same as the preparation process of compound **14a** in Example 1, except that: in step S9, the compound shown in Formula 13 was prepared by using 4-aminomethylpyridine instead of 2-aminomethylpyridine to finally afford the compound **14s** (41 mg, 65%). The chemical structural formula is as follows:

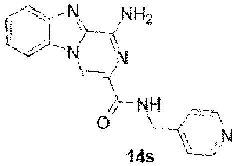

The characterization information of the product is as follows:
¹H NMR (500 MHz, DMSO-*d*₆) δ 8.95 (s, 1H), 8.80 (t, *J=* 6.1 Hz, 1H), 8.52 (d, *J=* 5.0 Hz, 2H), 8.44 (d, *J=* 8.2 Hz, 1H), 7.93 (d, *J=* 8.2 Hz, 1H), 7.57 (t, *J=* 7.6 Hz, 1H), 7.50-7.45 (m, 3H), 7.33 (d, *J*= 4.9 Hz, 2H), 4.58 (d, *J=* 6.2 Hz, 2H). ¹³C NMR (126 MHz, DMSO-*d*₆) δ 164.6, 150.0, 149.4, 148.9, 143.6, 136.3, 130.6, 130.1, 126.4, 123.6, 122.7, 120.7, 113.6, 111.7, 41.9.

### Example 20 Preparation of the 1-aminobenzo[4,5]imidazo[1,2-a]pyrazine-3-formamide compounds (Compound 14t)

The preparation process of compound **14t** was the same as the preparation process of compound **14a** in example 1, except that: in step S9, the compound shown in Formula 13 was prepared by using 2-furylmethylamine instead of 2-aminomethylpyridine to finally afford the compound **14t** (39 mg, 64%). The chemical structural formula is as follows:

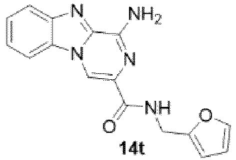

The characterization information of the product is as follows:
¹H NMR (500 MHz, DMSO-*d*₆) δ 8.95 (d, *J=* 1.4 Hz, 1H), 8.46 (d, *J=* 8.2 Hz, 1H), 8.42 (t, *J=* 5.9 Hz, 1H), 7.93 (d, *J=* 8.2 Hz, 1H), 7.63-7.62 (m, 1H), 7.59-7.55 (m, 1H), 7.52-7.47 (m, 3H), 6.44-6.43 (m, 1H), 6.36-6.35 (m, 1H), 4.58 (d, *J=* 6.0 Hz, 2H). ¹³C NMR (126 MHz, DMSO-*d*₆) δ 163.8, 152.4, 149.4, 143.6, 142.9, 136.2, 130.6, 130.0, 126.4, 123.6, 120.7, 113.6, 111.5, 111.0, 107.7, 36.1.

### Example 21 Preparation of the 1-aminobenzo[4,5]imidazo[1,2-a]pyrazine-3-formamide compounds (Compound 14u)

The preparation process of compound **14u** was the same as the preparation process of **14a** in Example 1, except that: in step S9, the compound shown in Formula 13 was prepared by using 1- (2-pyridyl)ethylamine instead of 2-aminomethylpyridine to finally afford the compound **14u** (44 mg, 66%). The chemical structural formula is as follows:

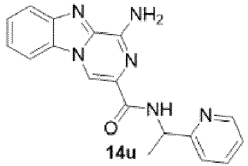

The characterization information of the product is as follows:
¹H NMR (500 MHz, Chloroform-*d*) δ 8.78-8.75 (m, 2H), 8.64 (d, *J=* 4.2 Hz, 1H), 7.98-7.92 (m, 2H), 7.68 (td, *J=* 7.6, 1.8 Hz, 1H), 7.58 (t, *J=* 7.7 Hz, 1H), 7.49 (t, *J=* 7.7 Hz, 1H), 7.35 (d, *J=* 7.8 Hz, 1H), 7.23-7.21 (m, 1H), 5.93 (s, 2H), 5.39 (p, *J=* 7.0 Hz, 1H), 1.64 (d, *J*= 6.9 Hz, 3H). ¹³C NMR (101 MHz, Chloroform-*d*) δ 163.2, 161.2, 149.4, 148.3, 143.6, 136.9, 135.8, 130.1, 129.9, 126.6, 123.8, 122.4, 121.5, 121.0, 111.7, 50.1, 22.4.

### Example 22 Preparation of the 1-aminobenzo[4,5]imidazo[1,2-a]pyrazine-3-formamide compounds (Compound 14v)

The preparation process of 14v was the same as the preparation process of compound 14a in Example 1, except that: in step S9, the compound shown in Formula 13 was prepared by using (quinoline-8-methyl)amine instead of 2-aminomethylpyridine to finally afford the compound 14v (48 mg, 65%). The chemical structural formula is as follows:

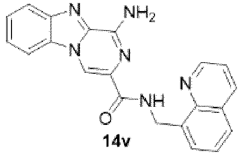

The characterization information of the product is as follows:
¹H NMR (500 MHz, DMSO-*d*₆) δ 9.06-9.05(m, 1H), 8.94 (s, 1H), 8.84 (t, *J=* 6.3 Hz, 1H), 8.45-8.42 (m, 2H), 7.93 (t, *J=* 9.1 Hz, 2H), 7.73 (d, *J=* 7.0 Hz, 1H), 7.64-7.54 (m, 3H), 7.49-7.46 (m, 3H), 5.16 (d, *J=* 6.3 Hz, 2H)

### Example 23 Preparation of the 1-aminobenzo[4,5]imidazo[1,2-a]pyrazine-3-formamide compounds (Compound 14w)

The preparation process of compound **14w** was the same as the preparation process of compound **14a** in Example 1, except that: in step S9, the compound shown in Formula 13 was prepared by using 1-(8-quinolinyl)ethylamine instead of 2-aminomethylpyridine to finally afford the compound **14 w** (52 mg, 68%). The chemical structural formula is as follows:

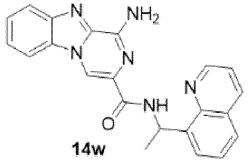

The characterization information of the product is as follows:
¹H NMR (500 MHz, DMSO-*d*₆) δ 9.47 (d, *J=* 9.0 Hz, 1H), 9.14 (d, *J=* 4.3 Hz, 1H), 8.88 (s, 1H), 8.44-8.41 (m, 2H), 7.94-7.91 (m, 2H), 7.80 (d, *J=* 7.1 Hz, 1H), 7.64-7.45 (m, 6H), 6.05 (p, *J*= 7.1 Hz, 1H), 1.69 (d, *J=* 6.6 Hz, 3H). ¹³C NMR (126 MHz, DMSO-*d*₆) δ 162.9 , 150.4, 149.4, 145.7, 143.6, 141.3, 137.3, 136.3, 130.6, 130.4, 128.9, 127.9, 127.3, 126.9, 126.4, 123.5, 121.9, 120.6, 113.6, 111.3, 47.4, 23.6.

### Example 24 Preparation of the 1-aminobenzo[4,5]imidazo[1,2-a]pyrazine-3-formamide compounds (Compound 14x)

The preparation process of compound **14x** was the same as the preparation process of compound **14a** in Example 1, except that: in step S9, the compound shown in Formula 13 was prepared by using 2-(pyridin-2-yl)-2-propylamine instead of 2-aminomethylpyridine to finally afford the compound **14x** (48 mg, 70%). The chemical structural formula is as follows:

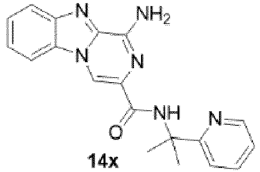

The characterization information of the product is as follows:
¹H NMR (400 MHz, Chloroform-*d*) δ 9.29 (s, 1H), 8.72 (s, 1H), 8.61 (d, *J=* 4.4 Hz, 1H), 7.96 (d, *J=* 8.3 Hz, 1H), 7.87 (d, *J=* 8.2 Hz, 1H), 7.72 (td, *J=* 7.9, 1.5 Hz, 1H), 7.57 (t, *J=* 7.6 Hz, 1H), 7.50-7.45 (m, 2H), 7.21-7.18 (m, 1H), 6.12 (s, 2H), 1.91 (s, 6H). ¹³C NMR (101 MHz, Chloroform-*d*) δ 164.6, 163.1, 148.3, 148.1, 143.7, 136.9, 135.8, 130.7, 130.1, 126.5, 123.7, 121.8, 121.0, 119.4, 111.6, 111.4, 57.0, 27.8.

### Example 25 Preparation of the 1-aminobenzo[4,5]imidazo[1,2-a]pyrazine-3-formamide compounds (Compound 14y)

The preparation process of compound **14y** was the same as the preparation process of compound **14a** in Example 1, except that: in step S9, the compound shown in Formula 13 was prepared by using 2-(3-chlorophenyl)ethylamine instead of 2-aminomethylpyridine to finally afford the compound **14y** (46 mg, 63%). The chemical structural formula is as follows:

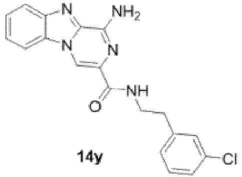

The characterization information of the product is as follows:
¹H NMR (500 MHz, DMSO-*d*₆) δ 8.89 (s, 1H), 8.43 (d, *J=* 8.0 Hz, 1H), 8.25 (d, *J=* 6.5 Hz, 1H), 7.91 (d, *J=* 7.9 Hz, 1H), 7.56 (t, *J=* 7.2 Hz, 1H), 7.49-7.45 (m, 3H), 7.35-7.32 (m, 2H), 7.28-7.23 (m, 2H), 3.60 (q, *J=* 7.0 Hz, 2H), 2.89 (t, *J=* 6.4 Hz, 2H). ¹³C NMR (126 MHz, DMSO-*d*₆) δ 164.0, 149.3, 143.6, 142.4, 136.2, 133.5, 130.7, 130.6, 130.2, 129.0, 127.9, 126.7, 126.4, 123.5, 120.6, 113.6, 111.3, 35.3.

### Example 26 Preparation of the 1-aminobenzo[4,5]imidazo[1,2-a]pyrazine-3-formamide compounds (Compound 14z)

The preparation process of 14z was the same as the preparation process of compound 14a in Example 1, except that: in step S9, the compound shown in the Formula 13 was prepared by using 2-(4-methoxyphenyl)ethylamine instead of 2-aminomethylpyridine to finally afford the compound 14z (51 mg, 72%). The chemical structural formula is as follows:

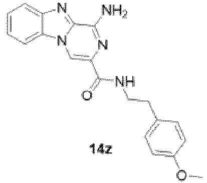

The characterization information of the product is as follows:
¹H NMR (500 MHz, DMSO-*d*₆) δ 8.90 (s, 1H), 8.44 (d, *J=* 8.2 Hz, 1H), 8.19 (t, *J=* 6.1 Hz, 1H), 7.92 (d, *J=* 8.2 Hz, 1H), 7.56 (t, *J=* 7.6 Hz, 1H), 7.50-7.46 (m, 3H), 7.19 (d, *J=* 8.5 Hz, 2H), 6.88 (d, *J=* 8.5 Hz, 2H), 3.73 (s, 3H), 3.55 (q, *J=* 6.9 Hz, 2H), 2.81 (t, *J=* 7.4 Hz, 2H). ¹³C NMR (126 MHz, DMSO-*d*₆) δ 163.9, 158.2, 149.3, 143.6, 136.2, 131.6, 130.6, 130.3, 130.1, 126.4, 123.5, 120.6, 114.3, 113.6, 111.2, 55.5, 40.9, 34.9.

### Example 27 Preparation of 1-aminobenzo[4,5]imidazo[1,2-a]pyrazine-3-formamide compounds (Compound 14aa)

The first part of the preparation process of compound **14aa** was the same as the preparation process of compound **14z** in Example 26, then compound **14z** was used as an intermediate and the reaction was carried out according to the following reaction formula, namely, methoxy group was converted to hydroxyl group by using boron tribromide. The specific steps were as follows: The compound shown in **14z** (36 mg, 0.1 mmol) was dissolved in 1 mL of dichloromethane and cooled to 0 °C, then a solution of boron tribromide in dichloromethane was added (0.2 mmol, 0.2 mL). The mixture was transferred to room temperature for reaction and then purified by column to finally afford the compound **14aa** (31 mg, 88%). The chemical structural formula is as follows:

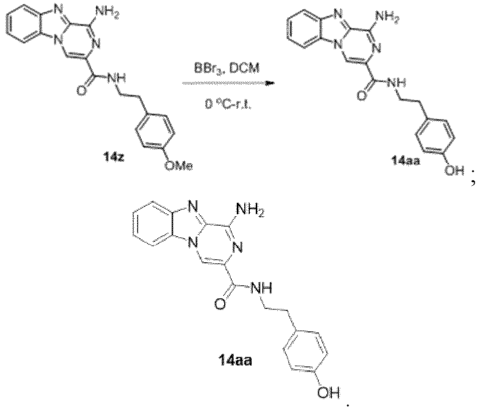

The characterization information of the product is as follows:
¹H NMR (500 MHz, DMSO-*d*₆) δ 9.24 (s, 1H), 8.91 (s, 1H), 8.44 (d, *J=* 8.1 Hz, 1H), 8.18 (t, *J=* 5.4 Hz, 1H), 7.92 (d, *J=* 8.1 Hz, 1H), 7.56 (t, *J=* 7.5 Hz, 1H), 7.50-7.48 (m, 3H), 7.07 (d, *J=* 8.0 Hz, 2H), 6.72 (d, *J=* 8.0 Hz, 2H), 3.53 (q, *J=* 6.4 Hz, 2H), 2.76 (t, *J=* 7.0 Hz, 2H). ¹³C NMR (126 MHz, DMSO-*d*₆) δ163.9, 156.2, 149.3, 143.6, 136.2, 130.6, 130.3, 130.0, 129.8, 126.4, 123.5, 120.6, 115.7, 113.6, 111.2, 41.0, 35.0.

### Example 28 Preparation of the 1-aminobenzo[4,5]imidazo[1,2-a]pyrazine-3-formamide compounds (Compound 14ab)

The preparation process of compound **14ab** was the same as the preparation process of compound **14a** in Example 1, except that: in step S9, the compound shown in the Formula 13 was prepared by using 2-aminomethylpyridine instead of 2-aminomethylpyridine to finally afford the compound **14ab** (52 mg, 69%). The chemical structural formula is as follows:

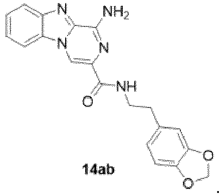

The characterization information of the product is as follows:
¹H NMR (500 MHz, DMSO-*d*₆) δ 8.90 (s, 1H), 8.44 (d, *J=* 8.2 Hz, 1H), 8.17 (t, *J=* 6.1 Hz, 1H), 7.92 (d, *J=* 8.2 Hz, 1H), 7.56 (t, *J=* 7.6 Hz, 1H), 7.49-7.45 (m, 3H), 6.86-6.83 (m, 2H), 6.72 (d, *J*= 7.7 Hz, 1H), 5.98 (s, 2H), 3.55 (q, *J=* 6.9 Hz, 2H), 2.80 (t, *J=* 7.3 Hz, 2H). ¹³C NMR (126 MHz, DMSO-*d*₆) δ 163.9, 149.3, 147.7, 146.0, 143.6, 136.2, 133.5, 130.6, 130.3, 126.4, 123.5, 122.0, 120.6, 113.6, 111.2, 109.4, 108.7, 101.2, 40.8, 35.5.

### Example 29 Preparation of the 1-aminobenzo[4,5]imidazo[1,2-a]pyrazine-3-formamide compounds (Compound 14ac)

The preparation process of compound **14ac** was the same as the preparation process of compound **14a** in Example 1, except that: in step S9, the compound shown in Formula 13 was prepared by using 2-(2-aminoethyl)pyridine instead of 2-aminomethylpyridine to finally afford the compound **14ac** (48 mg, 71%). The chemical structural formula is as follows:

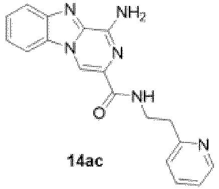

The characterization information of the product is as follows:
¹H NMR (500 MHz, DMSO-*d*₆) δ 8.90 (s, 1H), 8.58 (d, *J=* 4.8 Hz, 1H), 8.45-8.42 (m, 2H), 7.92 (d, *J=* 8.1 Hz, 1H), 7.73 (t, *J=* 7.7 Hz, 1H), 7.56 (t, *J=* 7.7 Hz, 1H), 7.49-7.46 (m, 3H), 7.32 (d, *J=* 7.8 Hz, 1H), 7.27-7.24 (m, 1H), 3.74 (q, *J=* 6.7 Hz, 2H), 3.05 (t, *J=* 7.1 Hz, 2H). ¹³C NMR (126 MHz, DMSO-*d*₆) δ 163.9, 159.6, 149.6, 149.3, 143.6, 137.1, 136.2, 130.6, 130.3, 126.4, 123.6, 123.5, 122.0, 120.6, 113.6, 111.2,38.8,37.6.

### Example 30 Preparation of the 1-aminobenzo[4,5]imidazo[1,2-a]pyrazine-3-formamide compounds (compound 14ad)

The preparation process of compound **14ad** was the same as the preparation process of compound 14a in Example 1, except that: in step S9, the compound shown in the Formula 13 was prepared by using 3-(2-aminoethyl)pyridine instead of 2-aminomethylpyridine to finally afford the compound **14ad** (46 mg, 69%). The chemical structural formula is as follows:

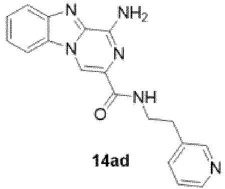

The characterization information of the product is as follows:
¹H NMR (500 MHz, DMSO-*d*₆) δ 8.90 (s, 1H), 8.49 (s, 1H), 8.44-8.43 (m, 2H), 8.26 (t, *J*= 6.2 Hz, 1H), 7.92 (d, *J=* 8.3 Hz, 1H), 7.71-7.69 (m, 1H), 7.56 (t, *J=* 6.7 Hz, 1H), 7.49-7.45 (m, 3H), 7.35-7.32 (m, 1H), 3.62 (q, *J=* 6.8 Hz, 2H), 2.91 (t, *J=* 7.2 Hz, 2H). ¹³C NMR (126 MHz, DMSO-*d*₆) δ 164.1, 150.3, 149.3, 148.0, 143.6, 136.7, 136.2, 135.3, 130.6, 130.2, 126.4, 124.0, 123.5, 120.6, 113.6, 111.3, 40.0, 32.9.

### Example 31 Preparation of the 1-aminobenzo[4,5]imidazo[1,2-a]pyrazine-3-formamide compounds (compound 14ae)

The preparation process of compound **14ae** was the same as the preparation process of compound **14a** in Example 1, except that: in step S9, the compound shown in Formula 13 was prepared by using 4-(2-aminoethyl)pyridine instead of 2-aminomethylpyridine to finally afford the compound **14ae** (39 mg, 59%). The chemical structural formula is as follows:

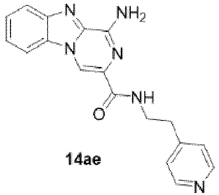

The characterization information of the product is as follows:
¹H NMR (500 MHz, DMSO-*d*₆) δ 8.89 (s, 1H), 8.49-8.48 (m, 2H), 8.43 (d, *J=* 8.3 Hz, 1H), 8.25 (t, *J*= 6.1 Hz, 1H), 7.92 (d, *J*= 8.2 Hz, 1H), 7.58-7.54 (m, 1H), 7.49-7.46 (m, 1H), 7.43 (s, 2H), 7.31-7.30 (m, 2H), 3.64 (q, *J=* 6.9 Hz, 2H), 2.92 (t, *J=* 7.2 Hz, 2H). ¹³C NMR (126 MHz, DMSO-*d*₆) δ 164.1, 150.0, 149.3, 148.8, 143.6, 136.2, 130.6, 130.2, 126.4, 124.7, 123.5, 120.6, 113.6, 111.3, 39.8, 34.9.

### Example 32 The inhibitory effect of 1-aminobenzo[4,5]imidazo[1,2-a]pyrazine-3-formamide compounds on adenosine A_{2A} receptors

Testing the binding of 1-aminobenzo[4,5]imidazo[1,2-a]pyrazine-3-formamide compounds in Examples 1-31 and the adenosine A_{2A} receptor. The specific process was as follows: A membrane protein solution (60 µg/tube, containing ADA 2 mg/mL) and a radioligand 1 nM [3H]-ZM241385 were added to each tube. Different compounds with the concentration of 10⁻⁴ M were added into the compound tubes respectively, shaken to mix uniformly, incubated at 37 °C for 30 minutes and the reaction was terminated in a water bath. Free and bound ligands were separated by suction filtration under negative pressure using GF/C glass fiber filter paper, washed three times with precooled 50 mM Tris-HCl, about 4 mL each time. Film was removed and placed upside down on the tray (i.e., with the grid side facing up), and baked for 3 minutes until dried. Dried small circular filter membrane was put into the liquid scintillation tube in sequence, and 2mL of scintillation solution was added. was provided with triple tubes, and the average value was taken. Finally, data analysis was carried out to calculate the inhibition rate of the compound on the adenosine A_{2A} receptor: Compound inhibition rate (I%) = (sum of compound cpm-compound cpm)/sum of compound cpm × 100%.

The test results are shown in Table 1. It can be seen from Table 1 that compounds **14a-14ae** have a good inhibitory effect on adenosine A_{2A} receptors. Among them, the inhibition rate on adenosine A_{2A} receptors of compounds **14a** to **14e**, compound **14k,** compound **14o**, compound **14p**, compounds **14r** to **14u**, compound **14w**, compound **14x** and compounds **14ac** to **14ae** is more than 70%. In particular, the inhibition rate of compounds **14a**, **14r**, **14s**, **14u** and **14ac** is more than 80%, and they show excellent inhibitory effect on adenosine A_{2A} receptor. They can be used as targeted adenosine A_{2A} receptor inhibitors for immunotherapy, by inhibiting the activity of adenosine A_{2A} receptor, releasing the elimination function of the immune system on tumor cells, thereby achieving the effect of treating tumors.

**Table 1 Test results of the activities of each compound on the adenosine A_{2A} receptor**

| Compound | Adenosine A_{2A} receptor inhibition rate | Compound | A_{2A} receptor inhibition rate |
|---|---|---|---|
| **14a** | 88.60±4.59% | 14r | 85.58±1.16% |
| **14b** | 71.00±4.02% | 14s | 85.04±2.94% |
| **14c** | 78.39±1.53% | 14t | 78.60±2.40% |
| **14d** | 75.55±1.46% | 14u | 86.45±1.02% |
| **14e** | 75.30±1.82% | 14v | 61.57±3.91% |
| **14f** | 65.56±4.23% | 14w | 78.27±2.31% |
| **14g** | 67.31±7.33% | 14x | 75.35±4.07% |
| **14h** | 64.91±4.24% | 14y | 47.61±5.81% |
| **14i** | 63.64±2.97% | 14z | 33.41±7.20% |
| **14j** | 62.22±7.34% | 14aa | 51.71±5.15% |
| **14k** | 73.15±1.62% | 14ab | 63.54±0.96% |
| **14l** | 63.75±6.80% | 14ac | 83.51±1.42% |
| **14m** | 69.25±7.46% | 14ad | 77.82±1.40% |
| **14n** | 68.28±5.53% | 14ae | 76.94±6.90% |
| **14o** | 79.68±2.94% | | |
| **14p** | 71.14±3.03% | | |
| **14q** | 48.89±5.72% | | |

The embodiments of the presented invention have been illustrated in detail above, but the presented invention is not limited to the described embodiments. For those skilled in the art, without departing from the principles and spirit of the present invention, various changes, modifications, substitutions, and variations made to these embodiments still falls within the protection scope of the present invention.

## Claims

1. A kind of 1-aminobenzo [4, 5] imidazo [1, 2-a] pyrazine-3-formamide compound, wherein, the structure of the compound is shown in formula (I): 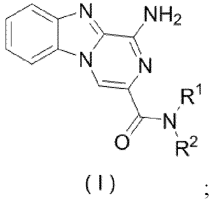
Wherein, R¹ is independently selected from hydrogen, C1-C4 alkyl, R² is independently selected from C1-C6 alkyl, C1-C4 alkanol group, C1-C4 haloalkyl, C1-C4 alkoxy, substituted phenyl, 2-pyridyl, 3-pyridyl, 4-pyridyl, 2-furyl, C4-C12 heterocycle, 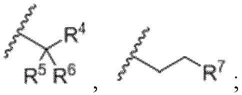
the said R⁴ is independently selected from substituted phenyl, 2-pyridyl, 3-pyridyl, 4-pyridyl, pyrimidinyl, pyrrolyl, imidazolyl, pyrazolyl, oxazolyl, pyrazinyl, pyridazinyl, 2-furyl, 2-substituted furyl, thienyl, 2-substituted thienyl, substituted indolyl, C6-C12 fused ring, C6-C12 N-hetero fused ring;
the said R⁵ is independently selected from hydrogen, deuterium, halogen, hydroxy, nitro, cyano, C1-C4 alkyl, C1-C4 haloalkyl, C1-C4 alkoxy, C3-C6 cycloalkyl;
the said R⁶ is independently selected from hydrogen, deuterium, halogen, hydroxy, nitro, cyano, C1-C4 alkyl, C1-C4 haloalkyl, C1-C4 alkoxy, C3-C6 cycloalkyl;
the said R⁷ is independently selected from substituted phenyl, 2-pyridyl, 3-pyridyl, 4-pyridyl, pyrimidinyl, pyrrolyl, imidazolyl, pyrazolyl, oxazolyl, pyrazine, pyridazinyl, 2-furyl, 2-substituted furyl, thienyl, 2-substituted thienyl, C6-C12 fused ring, C6-C12 N-hetero fused ring;
the said substituents in the 2-substituted furyl, substituted phenyl, and substituted indolyl are independently selected from C1-C4 alkyl, C1-C4 haloalkyl, C1-C4 alkoxy, C1-C4 alkanol group, cyano, halogen, hydroxyl and amino.

2. The 1-aminobenzo [4, 5] imidazo [1, 2-a] pyrazine-3-formamide compound according to claim 1, wherein, the said R¹ is independently selected from hydrogen, methyl, ethyl, propyl, isopropyl, butyl; R² is independently selected from n-pentyl, isopentyl, n-butyl, isobutyl, 2-methoxyethyl, 2,2-difluoroethyl, 2-hydroxyethyl, 1-hydroxypropyl, 2-hydroxypropyl, 3-hydroxypropyl, 3-hydroxybutyl, 2-pyridyl, 3-pyridyl, 4-pyridyl, 2-furyl, 2-substituted furyl, thienyl, 2-substituted thienyl, substituted phenyl, 2-pyridyl, 3-pyridyl, 4-pyridyl, pyrimidinyl, pyrrolyl,, imidazolyl, pyrazolyl, oxazolyl, pyrazinyl, pyridazinyl, quinolyl, isoquinolyl, naphthyl, indolyl, substitute indolyl, 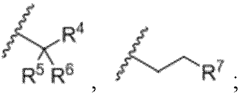
the said R⁴ is independently selected from substituted phenyl, 2-furyl, 2-substituted furyl, thienyl, 2-substituted thienyl, 2-pyridyl, 3-pyridyl, 4-pyridyl, pyrimidinyl, pyrrolyl, imidazolyl, pyrazolyl, oxazolyl, pyrazinyl, pyridazinyl, quinolyl, isoquinolyl, naphthyl, indolyl, substituted indolyl;
R⁵ is independently selected from hydrogen, deuterium, halogen, hydroxy, nitro, cyano, methyl, ethyl, propyl, isopropyl, trifluoromethyl, methoxy, ethoxy, cyclopropyl;
R⁶ is independently selected from hydrogen, deuterium, halogen, hydroxy, nitro, cyano, methyl, ethyl, propyl, isopropyl, trifluoromethyl, methoxy, ethoxy, cyclopropyl;
R⁷ is independently selected from substituted phenyl, thienyl, 2-substituted thienyl, 2-pyridyl, 3-pyridyl, 4-pyridyl, pyrimidinyl, pyrrolyl, imidazolyl, pyrazolyl, oxazolyl, pyrazinyl, pyridazinyl, 2-furyl, 2-substituted furyl, quinolyl, isoquinolyl, naphthyl, indolyl, substituted indolyl;
the said substituents in the 2-substituted furyl, substituted phenyl and substituted indolyl are C1-C4 alkyl, C1-C4 haloalkyl, C1-C4 alkoxy and C1-C4 alkanol group, cyano, halogen, hydroxyl and amino.

3. The 1-aminobenzo [4, 5] imidazo [1, 2-a] pyrazine-3-formamide compound according to claim 1, wherein, the compound is selected from any one of the following structural formulas:

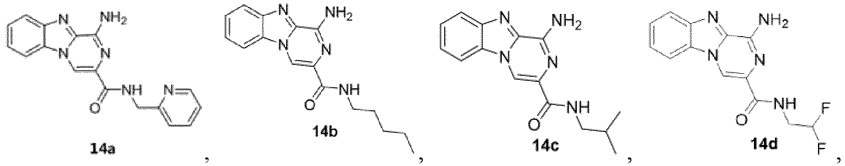

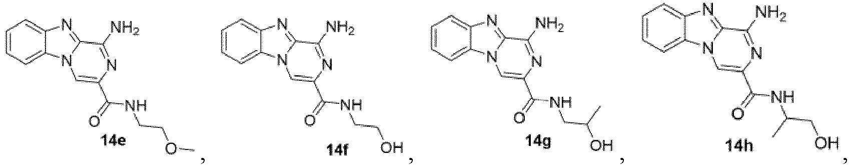

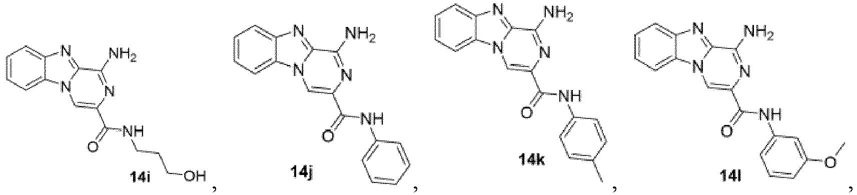

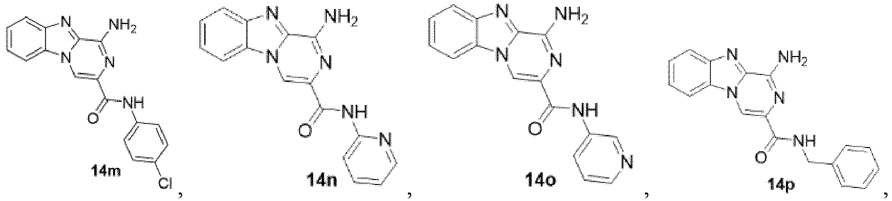

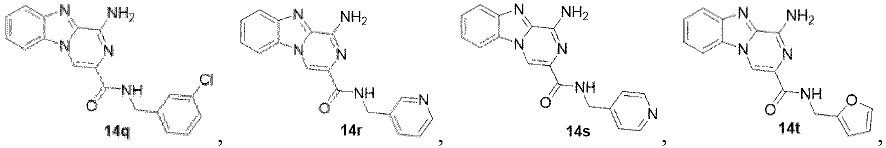

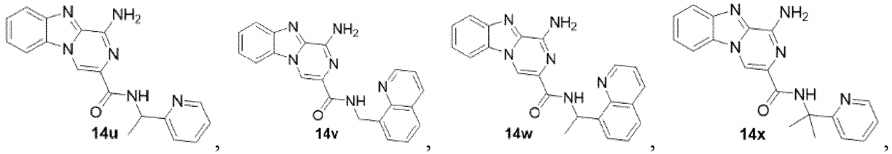

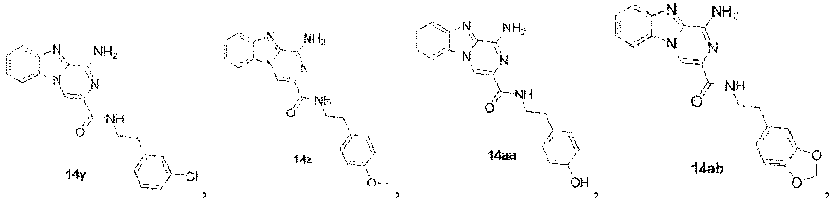

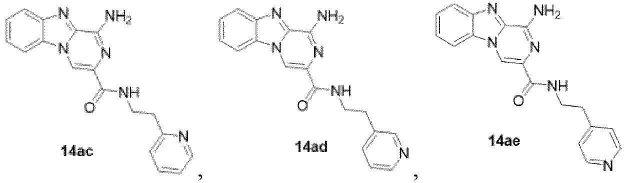

4. The method for the preparation of 1-aminobenzo [4, 5] imidazo [1, 2-a] pyrazine-3-formamide compound according to claim 1 or claim 2, wherein, comprises the following steps:
S1, according to the following reaction formula, the compound shown in the Formula 1 was dissolved in an acidic solvent and cooled to 0-10 °C, and then the compound shown in the Formula 2 was added dropwise, and then transferred to room temperature for reaction, to obtain the compound shown in the Formula 3: 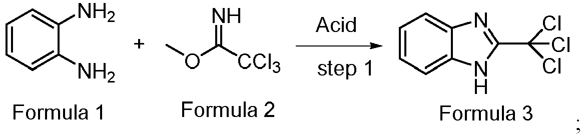
S2, according to the following reaction formula, the compound shpwn in the Formula 3 was cooled to 0-10 °C, and added the ammonia solution of 1, 4- dioxane, the reaction was carried out at room temperature under sealed condition to obtain the compound shown in the Formula 4: 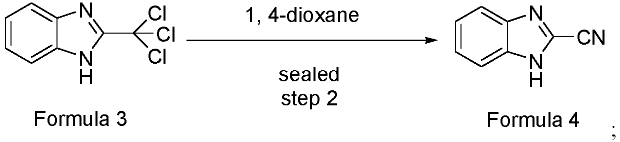
S3, according to the following reaction formula, the compound shown in the Formula 4 was dissolved in an alkaline organic solvent and cooled to -20-0 °C, and added the compound shown in the Formula 5, and then the reaction was carried out at this temperature to obtain the compound shown in the Formula 6: 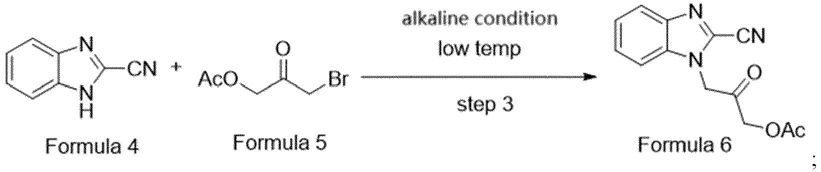
S4, according to the following reaction formula, the compound shown in the Formula 6 was mixed with ammonium salt and acid and the mixture was heated to 80-100 °C, and then the reaction was carried out at this temperature to obtain the compound shown in the Formula 7: 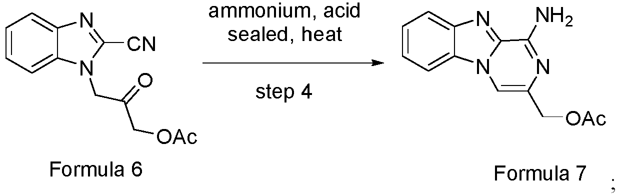
S5, according to the following reaction formula, the compound shown in the Formula 7 was dissolved in an alkaline organic solvent and the mixture was cooled to 0 °C, and added Di-tert-butyl dicarbonate, and then transferred to room temperature for reaction, to obtain the compound shown in the Formula 8: 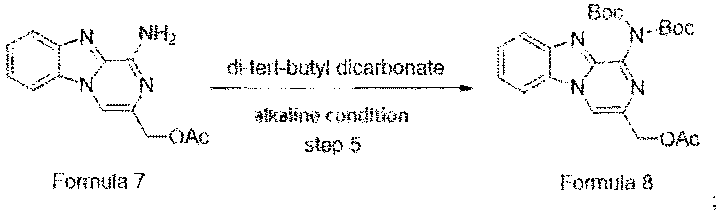
S6, according to the following reaction formula, the compound shown in the Formula 8 was dissolved in an organic solvent and cooled to 0 °C, and added the alkaline substance, and then transferred to room temperature for reaction to obtain the compound shown in the Formula 9: 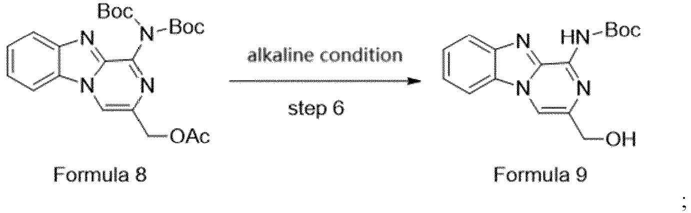
S7, according to the following reaction formula, the compound shown in Formula 9 was dissolved in organic solvent and cooled to 0 °C, and added the oxidizing agent, and then transferred to room temperature for reaction to obtain the compound shown in the Formula 10: 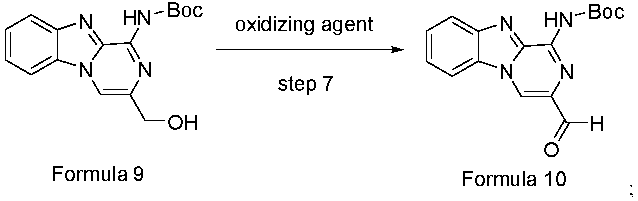
S8, according to the following reaction formula, the compound shown in the Formula 10 was dissolved in organic solvent and cooled to 0 °C and added the oxidizing agent, and then the reaction was carried out at this room temperature to obtain the compound shown in the Formula 11: 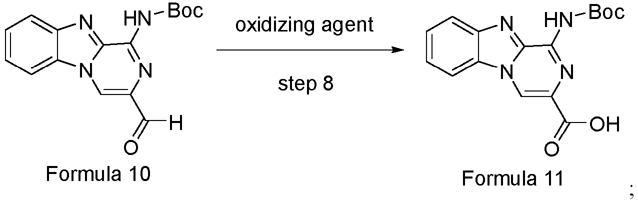
S9, according to the following reaction formula, the compounds shown in Formula 11 and Formula 12 were dissolved in organic solvent and cooled to 0 °C and added condensing agent, and then transferred to room temperature for reaction to obtain the compound shown in the Formula 13: 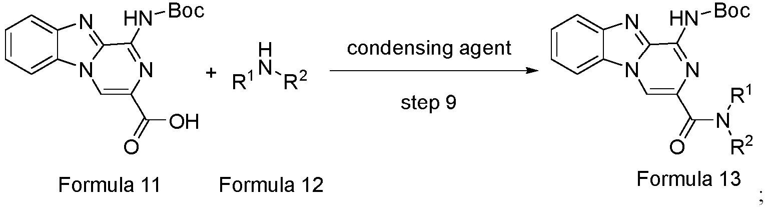
S10, according to the following reaction formula, the compound shown in the Formula 13 was dissolved in organic solvent and cooled to 0 °C, and added acidic substance, and then transferred to room temperature for reaction to obtain the target compound:

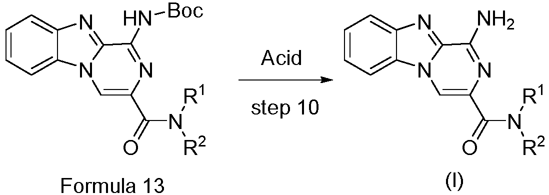

5. The method for the preparation of 1-aminobenzo [4, 5] imidazo [1, 2-a] pyrazine-3-formamide compound according to claim 3, wherein, in step S9, the compound shown in the Formula 12 is selected from 2-aminomethylpyridine, n-pentylamine, isobutylamine, 2,2-difluoroethylamine, 2-methoxyethylamine, ethanolamine, isopropanolamine, DL-aminopropanol, 3-amino-1-propanol, substitues 2-aminomethylpyridine, aniline, p-toluidine, m-aminoanisole, p-chlorobenzeneamine, 2-aminopyridine, 3-aminopyridine, benzylamine, 3-chlorobenzylamine, 3-aminomethylpyridine, 4-aminomethylpyridine, 2-furylmethylamine, 1- (2-)Pyridine) ethylamine substitues 2-aminomethylpyridine, (quinoline-8-methyl) amine, 1- (8-quinoline) ethylamine, 2- (pyridin-2-yl) -2-propylamine, 2- (3-chlorophenyl)ethylamine and 2- (4-methoxyphenyl) ethylamine.

6. The method for the preparation of 1-aminobenzo [4, 5] imidazo [1, 2-a] pyrazine-3-formamide compound according to claim 3, wherein, in step S9, the molar mass ratio of the compound shown in the Formula 11 to the compound shown in the Formula 12 is 1:1.

7. Application of the 1-aminobenzo [4, 5] imidazo [1, 2-a] pyrazine-3-formamide compound as described in the claims 1-3 in the preparation of the adenosine receptor inhibitor or anti-tumor drugs.

8. The application according to claim 7, wherein, the adenosine receptor is an adenosine A_{2A} receptor, the anti-tumor drug is a tumor immunotherapy drug targeting A_{2A} receptor.

9. An adenosine receptor inhibitor, wherein, the inhibitor uses the 1-aminobenzo [4, 5] imidazo [1, 2-a] pyrazine-3-formamide compound according to any one of claims 1-3 as the main active ingredient.

10. An anti-tumor drug, wherein, the drug uses the 1-aminobenzo [4, 5] imidazo [1, 2-a] pyrazine-3-formamide compound according to any one of claims 1-3 as the main active ingredient.
